(19) Europäisches Patentamt / European Patent Office / Office européen des brevets

(11) **EP 4 493 937 B1**

(12) **EUROPEAN PATENT SPECIFICATION**

(45) Date of publication and mention
of the grant of the patent:
**28.01.2026  Bulletin 2026/05**

(21) Application number: **23712019.1**

(22) Date of filing: **17.03.2023**

(51) International Patent Classification (IPC):
***G01N 33/68*** (2006.01)     ***G01N 33/92*** (2006.01)

(52) Cooperative Patent Classification (CPC):
**G01N 33/6893; G01N 33/92;** G01N 2333/4712;
G01N 2333/4722; G01N 2333/4737;
G01N 2333/495; G01N 2333/50; G01N 2333/51;
G01N 2333/515; G01N 2333/58; G01N 2800/324;
G01N 2800/60

(86) International application number:
**PCT/EP2023/056954**

(87) International publication number:
**WO 2023/175176 (21.09.2023 Gazette 2023/38)**

(54) **CMYBPC MARKER COMBINATIONS FOR EARLY DISCRIMINATION OF TYPE 2 VERSUS TYPE 1 ACUTE MYOCARDIAL INFARCTION**

CMYBPC-MARKER-KOMBINATIONEN ZUR FRÜHZEITIGEN UNTERSCHEIDUNG ZWISCHEN AKUTEM MYOKARDINFARKT TYP 2 UND TYP 1

COMBINAISONS DE MARQUEURS CMYBPC POUR LA DISCRIMINATION PRÉCOCE D'INFARCTUS AIGU DU MYOCARDE DE TYPE 1 PAR RAPPORT AU TYPE 2

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **18.03.2022  EP 22162968**

(43) Date of publication of application:
**22.01.2025  Bulletin 2025/04**

(73) Proprietor: **F. Hoffmann-La Roche AG**
**4070 Basel (CH)**

(72) Inventors:
• **BUEHLMANN, David**
  **6343 Rotkreuz (CH)**
• **DUEFEL, Hartmut**
  **82377 Penzberg (DE)**
• **EBERL, Magdalena**
  **82377 Penzberg (DE)**
• **GERG, Michael**
  **82377 Penzberg (DE)**
• **KASTNER, Peter**
  **82377 Penzberg (DE)**
• **KURTKAYA, Ulrike**
  **82377 Penzberg (DE)**
• **PIEHLER, Alexander**
  **82377 Penzberg (DE)**
• **WIENHUES-THELEN, Ursula-Henrike**
  **82377 Penzberg (DE)**
• **ZIEGLER, Andre**
  **6343 Rotkreuz (CH)**

(74) Representative: **Altmann Stößel Dick**
**Patentanwälte PartG mbB**
**Theodor-Heuss-Anlage 2**
**68165 Mannheim (DE)**

(56) References cited:
• **ANONYMOUS: "MemCode(TM) Reversible Protein Stain Kit - for PVDF Membranes Product 24585 - Thermo Scientific", MANUAL INSTRUCTIONS, 1 January 2010 (2010-01-01), XP055952056, Retrieved from the Internet <URL:https://assets.fishersci.com/TFS-Assets/ LSG/manuals/ MAN0011507_MemCode_Rev_Protein_ Stain_PVDFMemb_UG.pdf> [retrieved on 20220816]**

- NESTELBERGER THOMAS ET AL: "Cardiovascular Biomarkers in the Early Discrimination of Type 2 Myocardial Infarction", JAMA CARDIOLOGY, 6(7), 21 April 2021 (2021-04-21), pages 771 - 780, XP055951524, Retrieved from the Internet <URL:https://www.ncbi.nlm.nih.gov/pmc/articles/PMC8060883/> [retrieved on 20220815], DOI: 10.1001/jamacardio.2021.0669
- NESTELBERGER THOMAS ET AL: "Cardiovascular Biomarkers in the Early Discrimination of Type 2 Myocardial Infarction-Supplemental Online content", JAMA CARDIOLOGY, 6(7), 24 April 2021 (2021-04-24), pages 771 - 780, XP055952238, Retrieved from the Internet <URL:https://www.ncbi.nlm.nih.gov/pmc/articles/PMC8060883/bin/jamacardiol-e210669-s001.pdf> [retrieved on 20220817], DOI: 10.1001/jamacardio.2021.0669
- GOVINDAN SURESH ET AL: "Increase in cardiac myosin binding protein-C plasma levels is a sensitive and cardiac-specific biomarker of myocardial infarction", AM J CARDIOVASC DIS, 3(2), 15 June 2013 (2013-06-15), pages 60 - 70, XP055952072, Retrieved from the Internet <URL:https://www.ncbi.nlm.nih.gov/pmc/articles/PMC3683403/pdf/ajcd0003-0060.pdf> [retrieved on 20220816]
- KAIER THOMAS EDWARD ET AL: "DIRECT COMPARISON OF CARDIAC MYOSIN-BINDING PROTEIN C TO CARDIAC TROPONINS FOR THE EARLY DIAGNOSIS OF ACUTE MYOCARDIAL INFARCTION", JOURNAL OF THE AMERICAN COLLEGE OF CARDIOLOGY, vol. 69, no. 11, 21 March 2017 (2017-03-21), pages 220, XP029958399, ISSN: 0735-1097, DOI: 10.1016/S0735-1097(17)33609-4
- BAKER JAMES O ET AL: "Cardiac myosin-binding protein C: a potential early biomarker of myocardial injury", BASIC RESEARCH IN CARDIOLOGY, SPRINGER BERLIN HEIDELBERG, BERLIN/HEIDELBERG, vol. 110, no. 3, 3 April 2015 (2015-04-03), pages 1 - 14, XP035501729, ISSN: 0300-8428, [retrieved on 20150403], DOI: 10.1007/S00395-015-0478-5
- REINDL MARTIN ET AL: "FIBROBLAST GROWTH FACTOR 23 AS NOVEL BIOMARKER FOR EARLY RISK STRATIFICATION AFTER ST-ELEVATION MYOCARDIAL INFARCTION", JOURNAL OF THE AMERICAN COLLEGE OF CARDIOLOGY, ELSEVIER, AMSTERDAM, NL, 16 March 2017 (2017-03-16), XP085097645, ISSN: 0735-1097, DOI: 10.1016/S0735-1097(17)34868-4
- TAKAHASHI HIDEAKI ET AL: "Changes in Serum Fibroblast Growth Factor 23 in Patients With Acute Myocardial Infarction", CIRCULATION JOURNAL, VOL. 82, 1 March 2018 (2018-03-01), pages 767 - 774, XP055952253, Retrieved from the Internet <URL:https://www.jstage.jst.go.jp/article/circj/82/3/82_CJ-17-0826/_pdf> [retrieved on 20220817], DOI: 10.1253/circj.CJ-17-0826
- SKOWERSKI TOMASZ ET AL: "Angiopoietin-2 as a biomarker of non-ST-segment elevation myocardial infarction in patients with or without type 2 diabetes", ARCHIVES OF MEDICAL SCIENCE, 18(3), 7 November 2019 (2019-11-07), pages 624 - 631, XP055952301, Retrieved from the Internet <URL:https://www.ncbi.nlm.nih.gov/pmc/articles/PMC9102672/pdf/AMS-18-3-112327.pdf> [retrieved on 20220817], DOI: 10.5114/aoms.2019.89201
- WEI PENG ET AL: "The Relationship Between the Level of Serum ESM-1 and Lp-PLA2 in Patients With Acute ST-Segment Elevation Myocardial Infarction", CLINICAL AND TRANSLATIONAL SCIENCE, 14, 25 July 2020 (2020-07-25), pages 179 - 183, XP055952304, Retrieved from the Internet <URL:https://onlinelibrary.wiley.com/doi/full-xml/10.1111/cts.12838> [retrieved on 20220817], DOI: 10.1111/cts.12838
- CHUA WINNIE ET AL: "A MULTIPLE BLOOD BIOMARKER MODEL FOR IDENTIFYING PATIENTS WITH PREVALENT AF", HEART, 106 (SUPPL 2), 17 July 2020 (2020-07-17), pages A50 - A51, XP055952329, Retrieved from the Internet <URL:https://heart.bmj.com/content/heartjnl/106/Suppl_2/A50.2.full.pdf> [retrieved on 20220817]
- LIJUN SUN ET AL: "Bone Morphogenetic Protein-10 induces cardiomyocyte proliferation and improves cardiac function after myocardial infarction :", JOURNAL OF CELLULAR BIOCHEMISTRY, 1 June 2014 (2014-06-01), Hoboken, USA, pages n/a - n/a, XP055683699, ISSN: 0730-2312, DOI: 10.1002/jcb.24856

Remarks:
The complete document including Reference Table(s) and the Sequence Listing(s) can be downloaded from the EPO website

**Description**

The present invention relates to a method for assessing myocardial infarction comprising the steps of determining the amount of a first biomarker in a sample of a subject, said first biomarker being cMyBPC, determining the amount of a second biomarker in a sample of the subject, wherein said second biomarker is selected from the group consisting of: a BMP10-type peptide (Bone Morphogenic Protein 10-type peptide), FGF23 (Fibroblast growth factor 23), a BNP-type peptide (Brain natriuretic peptide type peptide), GDF-15 (Growth differentiation factor 15), ANG2 (Angiopoietin 2), CRP (C-reactive protein), ESM1 (endothelial cell specific molecule 1), or a lipid biomarker, such as Cholesterol or LDL (Low Density Lipoprotein), comparing the amounts of the biomarkers to references for said biomarkers and/or calculating a score for assessing myocardial infarction based on the amounts of the biomarkers, and assessing said subject based on the comparison and/or the calculation. The invention also relates to the use of a first biomarker being cMyBPC and a second biomarker selected from the group consisting of: a BMP10-type peptide, FGF23, a BNP-type peptide, GDF15, ANG2, CRP (C-reactive protein), ESM1, or a lipid biomarker, such as Cholesterol or LDL, or at least one detection agent for said first biomarker and at least one detection agent for said second biomarker for assessing myocardial infarction. Moreover, the invention further relates to a computer-implemented method for assessing myocardial infarction and a device and a kit for assessing myocardial infarction.

An aim of modern medicine is to provide personalized or individualized treatment regimens. Those are treatment regimens which take into account a patient's individual needs or risks. Personalized or individual treatment regimens shall be even taken into account for emergency measures where it is required to decide on potential treatment regimens within short periods of time. In such settings, the measurement of circulating biomarkers with a blood test can help to diagnose a disease state (such as injury of myocardial cells with a troponin test), to mechanistically understand the disease risk (e.g. elevated lipids and atherosclerosis) and to identify biological pathways involved (e.g. protective effects of BMP10 in atherosclerotic plaques; Upton PD et al., J Cell Sci 2020;133:jcs239715; doi:10.1242/jcs.239715), and helping to improve therapeutic effects.

The diagnostic workup of patients with suspected AMI (acute myocardial infarction) requires admission to an ED, registration of a 12-lead electrocardiogram (ECG), a blood test to diagnose or to exclude myocardial injury, assessment of clinical symptoms and history, physical examination, and other diagnostic tests for diagnosis of AMI or differential diagnoses.

According to the Universal Definition of AMI (Thygesen K et al., Circulation 2018;138:e618-e651. doi: 10.1161/CIR.0000000000000617.), five different types of AMI are defined based on the different mechanistic pathways underlying AMI, where Type-1 and Type 2 myocardial infarction are described in more detail below. According to the guidelines, AMI Types 3-5 are clinically differentiated well by the skilled person for 3 particular clinical situations such as Type 3 (defined as "death before it is possible to obtain blood for cardiac biomarker determination; or the patient may succumb soon after the onset of symptoms before an elevation of biomarker values has occurred"), Type 4 (PCI- and stent-related AMI), and Type 5 (CABG-related AMI). The present invention, therefore, aims at the most common situation of diagnosing suspected AMI (Types 1 and 2), with their differentiation (Type 1 vs Type 2) being very relevant to the choice of therapy:

Type 1 myocardial infarction (T1MI): Coronary atherothrombosis triggered by plaque rupture and plaque erosion in one or more coronary arteries and/or distal embolization, resulting in intraluminal thrombosis and subsequent decrease in myocardial perfusion and necrosis.

Type 2 myocardial infarction (T2MI): Occurs secondary to an acute imbalance of myocardial oxygen supply and demand mismatch. Reduced myocardial perfusion might be attributable to stable coronary atherosclerosis (without plaque rupture), coronary artery spasm, microvascular dysfunction, coronary embolism or dissection as well as impaired systemic hemodynamics including hypotension, hypertension, tachycardia, or hypoxemia (DeFilippis et al, Circulation 2019:140;1661-1678).

Type 2 MI tends to be more frequent in women and has been described to present higher short- and long term mortality rates than type 1 MI, as it is often associated with the presence of other underlying comorbidities (Thygesen et al, Eur Heart J 2018:40(3);246-247). Five-year survival rates of type 2 MI patients have been described to be as low as 40% or less (McCarthy et al, JAMA 2018:320(5);433).

Treatments of type 1 and type 2 myocardial infarction differ substantially as myocardial injury in type 2 MI is not due to atherosclerosis and unstable coronary artery disease (CAD), so that treatment should address the root and underlying cause (e.g. oxygen therapy in case of hypoxaemia or volume substitution in case of hypotension). This is in contrast to type 1 myocardial infarction, where, depending on the degree of infarction and availability of percutaneous coronary intervention (PCI), immediate invasive treatment or fibrinolysis is necessary. In case of type 2 MI, coronary examination may be useful to determine whether or not underlying CAD is present (Thygesen et al, Eur Heart J 2018:40(3);246-247). Default treatment of a type 2 MI with specific therapies for a type 1 MI is not currently supported by clinical evidence and may even influence the outcome unfavorably (Collinson et al, ACC May 18, 2016, Diagnosing Type 2 Myocardial Infarction), hence making this distinction all the more crucial. While troponin peak levels tend to be higher in T1 MI vs T2 MI, the discriminatory power in a clinical setting remains insufficient (Smilowitz et al, Coronary Artery Disease 2018;29(1);46-52). Therefore, it is an important unmet need to identify and differentiate patients with type 2 versus type 1 MI (Nagele 2020 Circulation. 2020;141:1431-1433. DOI: 10.1161/CIRCULATIONAHA. 119.044996).

cMyBPC is the cardiac isoform of MyBP and is a structural muscle protein of cardiac myocytes. MYBP-C has a high cardiac specificity and is released into blood stream following cardiac muscle necrosis. Elevated concentrations of cardiac muscle necrosis biomarkers were described in blood samples of patients with acute myocardial infarction. Cardiac muscle necrosis biomarkers including troponins and cMyBPC were observed in differential concentrations in type 1 MI versus type 2 MI patients, see e.g. Nestelberger et al., 2021 JAMA Cardiol. doi:10.1001/jamacardio.2021.0669.

However, cardiac muscle necrosis biomarkers including troponins and cMyBPC were described in the study of Nestelberger et al. (2021) to show only moderate clinical performance.

Therefore, it is an important unmet need to identify and differentiate patients with type 2 versus type 1 MI (Nagele 2020 Circulation. 2020;141:1431-1433. DOI: 10.1161/CIRCULATIONAHA. 119.044996).

The present invention, therefore, provides means and methods complying with these needs.

Advantageously, it has been found in the studies underlying the present invention that a combination of the biomarker cMyBPC with a second and, preferably, a third biomarker allows for a reliable and early assessment of myocardial infarction. In the studies, patients presenting at emergency departments were investigated. To this end, patients with confirmed myocardial infarction were subdivided into those patients suffering from type 1 myocardial infarction and type 2 myocardial infarction. The amount of various biomarkers has been determined and the biomarkers were analyzed and mathematically combined via logistic regression analysis. The area under the receiver operating characteristic (AUC) was used to evaluate biomarker performance. The AUC values are the mathematical integer of a function f(x) within the interval [a][b]. AUC was also investigated for biomarker pairs and triplets. Biomarker combinations which together showed improved AUC over the best single biomarker AUC were identified. The results are described in the accompanying Examples, below.

Advantageously, it was shown in the studies underlying the present invention that combinations of cMyBPC with a BMP10-type peptide (Bone Morphogenic Protein 10-type peptide), FGF23 (Fibroblast growth factor 23), a BNP-type peptide (Brain natriuretic peptide type peptide), GDF-15 (Growth differentiation factor 15), ANG2 (Angiopoietin 2), CRP (C-reactive protein), ESM1 (endothelial cell specific molecule 1), or a lipid biomarker, such as Cholesterol or LDL (Low Density Lipoprotein) significantly improved the differentiation between T1MI and T2MI as compared to cMyBPC as a single marker. The best improvements were obtained for BMP10-type peptides (such as NT-proBMP10), FGF23 and BNP-type peptides (such as NT-proBNP.

Moreover, it was shown that the addition of a third biomarker could further improve the differentiation. For example, the combination of cMyBPC and a BMP 10-type peptide with a lipid biomarker or with CRP allowed for an improved assessment. Table 2 in the Examples section shows the results for different lipid biomarkers (Cholesterol, TAG, LDL (Low-density Lipoprotein), HDL (High-density Lipoprotein) and Apolipoprotein A-1).

Further, the addition of lipid biomarkers, such as CHOL, LDL or HDL, to a combination of cMyBPC and FGF23 allowed for an improved differentiation.

Moreover, it was shown in the studies underlying the present invention that combinations of cMyBPC and ANG2 are advantageous in patients with diabetes.

Further advantageous marker combinations of the invention can be found in Tables 2 and 5 in the Examples section.

In particular, if patients with suspected myocardial infarction are presenting in, e.g., emergency units, an early assessment of the patient is decisive to start therapeutic measures including drug administration, physical or other therapeutic interventions. In particular, it is important to differentiate between type 1 myocardial infarction and type 2 myocardial infarction since treatments of type 1 and type 2 myocardial infarction differ substantially (see above). Thanks to the present invention, life-threatening developments can be prevented because the identification of AMI patients can be assessed by biomarker determinations at an early stage, which is crucial to prevent myocardial damage (Collet JP et al, Eur Heart J 2021;42:1289-1367. doi: 10.1093/eurheartj/ehaa575). The biomarker pairs and triplets identified in the studies underlying the present invention are a reliable basis for medical decisions and the assessment can be performed in a time- and cost-effective manner.

The present invention relates to a method for assessing myocardial infarction in a subject:

(a) determining the amount of a first biomarker in a sample of the subject, said first biomarker being cMyBPC;
(b) determining the amount of a second biomarker in a sample of the subject, said second biomarker being a BMP10-type peptide (Bone Morphogenic Protein 10-type peptide), FGF23 (Fibroblast growth factor 23), a BNP-type peptide (Brain natriuretic peptide type peptide), GDF-15 (Growth differentiation factor 15), ANG2 (Angiopoietin 2), CRP (C-reactive protein), ESM1 (endothelial cell specific molecule 1), or a lipid biomarker, such as Cholesterol, or LDL (Low Density Lipoprotein);
(c) comparing the amounts of the biomarkers to references for said biomarkers and/or calculating a score for assessing myocardial infarction based on the amounts of the biomarkers; and

(d) assessing myocardial infarction based on the comparison and/or the calculation made in step (c), wherein the assessment of myocardial infarction is i) the differentiation between type 1 and type 2 myocardial infarction, ii) the diagnosis of type 2 myocardial infarction, or iii) the guidance of myocardial infarction therapy.

In an embodiment of method of the present invention, the method further comprises the determination of the amount of third biomarker. In particular, in step (b) of the method of the invention

(i) if the amount of a BMP10-type peptide is determined as the second biomarker, the method may further comprise determining the amount of CRP or at least one lipid biomarker selected from the group consisting of Cholesterol, TAG (Triglycerides), LDL (Low-density Lipoprotein), HDL (High-density Lipoprotein) and Apolipoprotein A-1; or

(ii) if the amount of FGF23 is determined as the second biomarker, the method may further comprise determining the amount of a BMP10-type peptide, a BNP-type peptide, CRP, ANG2, or at least one lipid biomarker selected from the group consisting of Cholesterol, TAG (Triglycerides), LDL and HDL as a third biomarker, or

(iii) if the amount of a BNP-type peptide is determined as the second biomarker, the method may further comprise determining the amount of Cholesterol or ANG2, or

(iv) if the amount of ANG-2 is determined as the second biomarker, the method may further comprise determining the amount of APOAT or LDL as a third biomarker.

Accordingly, the present invention relates to a method for assessing myocardial infarction in a subject:

(a) determining the amount of a first biomarker in a sample of the subject, said first biomarker being cMyBPC,

(b) determining the amount of a second and a third biomarker as set forth above in a (the) sample of the subject,

(c) comparing the amounts of the biomarkers to references for said biomarkers and/or calculating a score for assessing myocardial infarction based on the amounts of the biomarkers; and

(d) assessing myocardial infarction based on the comparison and/or the calculation made in step (c) as defined in the claims.

It is to be understood that as used in the specification and in the claims, "a" or "an" can mean one or more, depending upon the context in which it is used. Thus, for example, reference to "an" item can mean that at least one item can be utilized.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements. The term "comprising" also encompasses embodiments where only the items referred to are present, i.e. it has a limiting meaning in the sense of "consisting of".

Further, as used in the following, the terms "particularly", "more particularly", "typically", and "more typically" or similar terms are used in conjunction with additional / alternative features, without restricting alternative possibilities. Thus, features introduced by these terms are additional / alternative features and are not intended to restrict the scope of the claims in any way. The invention may, as the skilled person will recognize, be performed by using alternative features. Similarly, features introduced by "in an embodiment of the invention" or similar expressions are intended to be additional / alternative features, without any restriction regarding alternative embodiments of the invention, without any restrictions regarding the scope of the invention and without any restriction regarding the possibility of combining the features introduced in such way with other additional / alternative or non-additional / alternative features of the invention.

Further, it will be understood that the term "at least one" as used herein means that one or more of the items referred to following the term may be used in accordance with the invention. For example, if the term indicates that at least one sampling unit shall be used this may be understood as one sampling unit or more than one sampling units, i.e. two, three, four, five or any other number. Depending on the item the term refers to, the skilled person understands as to what upper limit the term may refer, if any.

The term "about" as used herein means that with respect to any number recited after said term an interval accuracy exists within in which a technical effect can be achieved. Accordingly, "about" as referred to herein, preferably, refers to the precise numerical value or a range around said precise numerical value of $\pm 20$ %, preferably $\pm 15$ %, more preferably $\pm 10$ %, or even more preferably $\pm 5$ %.

Furthermore, the terms "first", "second", "third" and the like in the description and in the claims are used for distinguishing between similar elements and not necessarily for describing a sequential or chronological order. For example, the amount of the second biomarker may be determined prior to the amount of the first biomarker.

The method of the present invention may consist of the aforementioned step or may comprise additional steps, such as steps for further evaluation of the assessment obtained in step (d), steps recommending or initiating therapeutic measures such as treatments, or the like. Moreover, it may comprise steps prior to step (a) such as steps relating to sample pretreatments. However, preferably, it is envisaged that the above-mentioned method is an ex vivo method which does not require any steps being practiced on the human or animal body. Thus, the method shall be an *in vitro* method. Moreover, the method may be assisted by automation. Typically, the determination of the biomarkers may be supported by robotic equipment while the comparison and assessment may be supported by data processing equipment such as computers.

In accordance with the present invention, myocardial infarction shall be assessed. The term "myocardial infarction" is well-known in the art. As used herein, the term refers to an acute myocardial infarction (abbreviated "AMI"). Clinical criteria of an acute myocardial infarction denote the presence of acute myocardial injury (i.e. cardiac troponin (cTn) value above the 99th percentile upper reference limit (URL) including rise and/or fall of cTn values) in the setting of evidence of acute myocardial ischemia. Evidence of acute myocardial ischemia include clinical signs and symptoms, ischemic ECG changes, development of pathological Q waves and imaging evidence of new loss of viable myocardium or new regional wall motion abnormality in a pattern consistent with an ischemic aetiology (Thygesen et al, Eur J Heart 2019:40(3);237-269, DOI:10.1093/eurheartj/ehy462).

In an embodiment of the present invention, the term "assessing myocardial infarction" relates to the differentiation between type 1 and type 2 myocardial infarction. Accordingly, it is determined whether a subject suffers from the type 1 or type 2 myocardial infarction.

Accordingly, the present invention relates to a method for differentiating between type 1 and type 2 myocardial infarction:

> (a) determining the amount of a first biomarker in a sample of the subject, said first biomarker being cMyBPC,
> (b) determining the amount of a second biomarker and, optionally, a third biomarker according to the claims in a (the) sample of the subject,
> (c) comparing the amounts of the biomarkers to references for said biomarkers and/or calculating a score for differentiating between type 1 and type 2 myocardial infarction based on the amounts of the biomarkers; and
> (d) differentiating between type 1 and type 2 myocardial infarction based on the comparison and/or the calculation made in step (c).

The term "type 1 myocardial infarction" is well-known in the art. As used herein, the term refers to an AMI with the underlying ischemic etiology of an atherosclerotic plaque rupture/erosion with occlusive or non-occlusive thrombus formation in one or multiple coronary arteries. Criteria for type 1 AMI therefore include previously mentioned criteria of an AMI including, for example, identification of a coronary thrombus by angiography including intracoronary imaging or autopsy as evidence of acute myocardial ischemia (Thygesen et al, Eur J Heart 2019:40(3);237-269, DOI:10.1093/eurheartj/ehy462).

The term "type 2 myocardial infarction" is well-known in the art. As used herein, the term refers to an AMI with the underlying ischemic etiology of an impaired oxygen supply-demand balance due to a) an increase in oxygen demand (e.g. sustained tachyarrhythmias or severe hypertension), or b) due to a decrease in oxygen supply (e.g. hypotension, bradyarrhythmias, respiratory failure, severe anemia, coronary vasospasms, coronary artery dissection or microvascular dysfunction). Accordingly, criteria for a type 2 AMI include previously mentioned criteria of an AMI including evidence of an imbalance between myocardial oxygen supply and demand unrelated to acute coronary athero-thrombosis (Thygesen et al, Eur J Heart 2019:40(3);237-269, DOI:10.1093/eurheartj/ehy462).

In another embodiment of the present invention, the term "assessing myocardial infarction" relates to the diagnosis of type 2 myocardial infarction.

Accordingly, the present invention relates to a method for diagnosing type 2 myocardial infarction:

> (a) determining the amount of a first biomarker in a sample of the subject, said first biomarker being cMyBPC,
> (b) determining the amount of a second biomarker and, optionally, a third biomarker according to the claims in a (the) sample of the subject,
> (c) comparing the amounts of the biomarkers to references for said biomarkers and/or calculating a score for diagnosing type 2 myocardial infarction based on the amounts of the biomarkers; and
> (d) diagnosing type 2 myocardial infarction based on the comparison and/or the calculation made in step (c).

The term "diagnosing" as used herein means assessing whether a subject as referred to in accordance with the method of the present invention suffers from type 2 myocardial infarction, or not.

In another embodiment of the present invention, the term "assessing myocardial infarction" relates to the assessment of a therapy for myocardial infarction or the treatment of myocardial infarction, preferably, in a subject suffering. Based on the present invention, treatment decisions can be made and the subject can be treated accordingly. For example, it can be decided whether a patient is subjected to a treatment that aims to treat type 2 myocardial infarction or to a treatment that aims to treat type 1 myocardial infarction.

The term "diagnosing" as used herein means assessing whether a subject as referred to in accordance with the method of the present invention suffers from type 2 myocardial infarction, or not.

As will be understood by those skilled in the art, the assessment, such as the differentiation or the diagnosis, made in accordance with the present invention, although preferred to be, may usually not be correct for 100% of the investigated subjects. The term, typically, requires that a statistically significant portion of subjects can be correctly assessed. Whether a portion is statistically significant can be determined without further ado by the person skilled in the art using various well known statistic evaluation tools, e.g., determination of confidence intervals, p-value determination, Student's t-test, Mann-Whitney test, etc.. Details may be found in Dowdy and Wearden, Statistics for Research, John Wiley & Sons, New York 1983. Typically envisaged confidence intervals are at least 50%, at least 60%, at least 70%, at least 80%, at least 90%, at least 95%. The p-values are, typically, 0.2, 0.1, 0.05.

In accordance with the present invention, the assessment of myocardial infarction is typically understood as an aid in the assessment of myocardial infarction, e.g. as an aid in differentiating between type 2 and type 1 myocardial infarction, or as an aid in diagnosing type 2 myocardial infarction. Thus, the methods and uses of the present invention may be part of a complete assessment. The complete assessment may include the assessment of further markers, clinical parameters and/or ECG (which e.g. may contribute to the score). The final assessment (such as the differentiation), in principle, will be carried out by physician.

The term "subject" as used herein refers to an animal, preferably a mammal and, more typically to a human. The subject to be investigated by the method of the present invention shall be suspected to suffer from myocardial infarction or shall suffer from myocardial infarction. Preferably, the subject suffers from myocardial infarction, such as from non ST-elevation myocardial infarction (NSTEMI). In an embodiment, the subject is male. In another embodiment, the subject is female.

However, the subject to be tested, preferably, does not suffer from Type 3, Type 4 or Type 5 myocardial infarction. An overview on these types of AMI can be found in Thygesen K et al.)Circulation 2018;138:e618-e651. doi: 10.1161/CIR.0000000000000617.) which herewith is incorporated by reference. Type 3 (defined as "death before it is possible to obtain blood for cardiac biomarker determination; or the patient may succumb soon after the onset of symptoms before an elevation of biomarker values has occurred"), Type 4 (PCI- and stent-related AMI), and Type 5 (CABG-related AMI). Thus, the subject shall suffer from type 1 or type 2 myocardial infarction. Alternatively, the subject shall be suspected to suffer from type 1 or type 2 myocardial infarction.

In a preferred embodiment, the subject is a diabetes patient, i.e. suffers from diabetes. For example, the subject may suffer from type 1 or type 2 diabetes. Typically, the diabetic subject suffers from type 2 diabetes. Table 5 in the Examples shows preferred markers and marker combinations for assessing patients suffering from diabetes, such as combinations of cMyBPC with ANG2. Thus, the second biomarker for diabetes patients is preferably ANG2.

The term "sample" as used herein refers to any sample that under physiological conditions comprises the first, second and/or third biomarkers referred to herein. More typically, the sample is a body fluid sample, e.g. a blood sample or sample derived therefrom (e.g. serum or plasma), a urine sample, interstitial fluid, a saliva sample a lymphatic fluid sample or the like. Most typically, said sample is a blood, serum or plasma sample.

Further, it is envisaged that a blood sample is a dried blood spot sample. Dried blood spot samples can be obtained by applying drops of blood onto absorbent filter paper. The blood is allowed to thoroughly saturate the paper and is air dried for several hours. The blood may have been drawn by a lancet from the subject to be tested, e.g. from the finger.

In a preferred embodiment, the sample is a blood (i.e. whole blood), serum or plasma sample. Serum is the liquid fraction of whole blood that is obtained after the blood is allowed to clot. For obtaining the serum, the clot is removed by centrifugation and the supernatant is collected. Plasma is the acellular fluid portion of blood. For obtaining a plasma sample, whole blood is collected in anticoagulant-treated tubes (e.g. citrate-treated or EDTA-treated tubes). Cells are removed from the sample by centrifugation and the supernatant (i.e. the plasma sample) is obtained.

Blood samples include capillary blood samples. Such samples can be obtained, e.g., from a puncture on the finger.

Preferably, the test subject suffers from myocardial infarction at the time at which the sample is obtained, although the subject might not have been diagnosed at that time to suffer from myocardial infarction. The diagnosis might be done later (e.g. based on the determination of a cardiac Troponin in a second sample obtained from the subject one to three hours after the first sample). How to diagnose myocardial infarction is well known in the art and e.g. described by Collet JP et al. (ESC Guidelines for the management of acute coronary syndromes in patients presenting without persistent ST-segment elevation. Eur Heart J. 2020 Aug 29:ehaa575. doi: 10.1093/eurheartj/ehaa575. Epub ahead of print. PMID: 32860058). Currently available protocols for the diagnosis of Non ST elevation ACS combine biomarker concentrations at presentation and at later time points, such as after 1 hours, 2 hours, or 3 hours.

Advantageously, the method of the present invention allows for the early assessment of myocardial infarction.

The assessment as referred to herein can be reliably made based on the amounts of the first, second and third biomarker in a sample (preferably a single sample) obtained from the subject at presentation. In an embodiment, the sample is thus a sample which has been obtained from a subject at presentation at the emergency department.

In accordance with the invention, the amount of at least three biomarkers shall be determined in a sample from the subject (preferably in a single sample) obtained at presentation: a first biomarker, a second biomarker, and, optionally, a third biomarker. In the following, combinations of the first, the second and, optionally the third biomarker are disclosed. The combinations apply to the entire subject-matter disclosed herein, such as the methods, uses, devices etc. of the present invention.

Biomarker combinations

The first marker is cMyBPC.

The second marker shall be at least one biomarker selected from the group consisting of a BMP10-type peptide, FGF23, a BNP-type peptide, GDF15, ANG2, CRP (C-reactive protein), ESM1, or a lipid biomarker, such as Cholesterol or LDL.

In an embodiment, the second biomarker is a BMP10-type peptide-type peptide (Bone Morphogenic Protein 10-type peptide), such as N-terminal proBMP10 or proBMP10.

In an alternative embodiment, the second biomarker is FGF23.

In an alternative embodiment, the second biomarker is a BNP-type peptide. Preferably, the BNP-type peptide is NT-proBNP, proBNP or BNP, more preferably NTproBNP or BNP, and most preferably NT-proBNP.

The BNP-type peptide may be glycosylated, or not (as described elsewhere herein).

In an alternative embodiment, the second biomarker is ANG2. Combinations with ANG2 can be advantageously used in diabetic patients (but are not limited to such patients).

In an alternative embodiment, the second biomarker is GDF15.

In an alternative embodiment, the second biomarker is a CRP, such as hsCRP.

In an alternative embodiment, the second biomarker is ESM1.

In an alternative embodiment, the second biomarker is a lipid biomarker.

For example, the lipid biomarker (as second marker) may be Cholesterol. Alternatively, the lipid biomarker may be LDL.

Moreover, a third biomarker may be used. The choice of the third biomarker may depend on the second biomarker.

For example, if the amount of a BMP10-type peptide is determined as the second biomarker, the method may, in an embodiment, further comprise determining the amount of CRP, ANG2 or at least one lipid biomarker selected from the group consisting of Cholesterol, TAG (Triglycerides), LDL (Low-density Lipoprotein), HDL (High-density Lipoprotein) and Apolipoprotein A-1. As shown in the Examples, the use of lipid biomarkers in combination with a BMP10-type peptide improved the assessment of the patient.

If the amount of FGF23 is determined as the second biomarker, the method may, in an embodiment, comprise determining the amount of a BMP10-type peptide, a BNP-type peptide, CRP, ANG2, or at least one lipid biomarker selected from the group consisting of Cholesterol, TAG (Triglycerides), LDL and HDL as a third biomarker.

If the amount of a BNP-type peptide is determined as the second biomarker, the method further comprises determining the amount of Cholesterol or ANG2 a third biomarker.

If the amount of ANG2 is determined as the second biomarker, the method may, in an embodiment, comprise determining the amount of APOAT or LDL a third biomarker.

Thus, the present invention envisages the following combinations of two or three markers.

In an embodiment, the first marker is cMyBPC, and the second marker is a BMP10-type peptide.

In an alternative embodiment, the first marker is cMyBPC, and the second marker is FGF23.

In an alternative embodiment, the first marker is cMyBPC, and the second marker is NTproBNP or BNP, in particular NT-proBNP.

In an alternative embodiment, the first marker is cMyBPC, and the second marker is tNTproBNP.

In an alternative embodiment, the first marker is cMyBPC, and the second marker is GDF15.

In an alternative embodiment, the first marker is cMyBPC, and the second marker is ANG2.

In an alternative embodiment, the first marker is cMyBPC, and the second marker is CHOL.

In an alternative embodiment, the first marker is cMyBPC, and the second marker is ESM1.

In an alternative embodiment, the first marker is cMyBPC, and the second marker is CRP, in particular hsCRP.

In an alternative embodiment, the first marker is cMyBPC, and the second marker is LDL.

In an alternative embodiment, the first marker is cMyBPC, the second marker is a BMP10-type peptide and the third marker is CHOL.

In an alternative embodiment, the first marker is cMyBPC, the second marker is a BMP10-type peptide and the third marker is LDL.

In an alternative embodiment, the first marker is cMyBPC, the second marker is a BMP10-type peptide and the third marker is TRIGL.

In an alternative embodiment, the first marker is cMyBPC, the second marker is a BMP10-type peptide and the third marker is CRP.

In an alternative embodiment, the first marker is cMyBPC, the second marker is a BMP10-type peptide and the third marker is APOAT.

In an alternative embodiment, the first marker is cMyBPC, the second marker is a BMP10-type peptide and the third marker is ANG2.

In an alternative embodiment, the first marker is cMyBPC, the second marker is a BMP10-type peptide and the third marker is HDL.

In an alternative embodiment, the first marker is cMyBPC, the second marker is FGF23 and the third marker is TRIGL.

In an alternative embodiment, the first marker is cMyBPC, the second marker is FGF23 and the third marker is a BMP10-type peptide.

In an alternative embodiment, the first marker is cMyBPC, the second marker is FGF23 and the third marker is NTproBNP or BNP, in particular NT-proBNP.

In an alternative embodiment, the first marker is cMyBPC, the second marker is FGF23 and the third marker is tNTproBNP.

In an alternative embodiment, the first marker is cMyBPC, the second marker is FGF23 and the third marker is CHOL.

In an alternative embodiment, the first marker is cMyBPC, the second marker is FGF23 and the third marker is HDL.

In an alternative embodiment, the first marker is cMyBPC, the second marker is FGF23 and the third marker is LDL.

In an alternative embodiment, the first marker is cMyBPC, the second marker is FGF23 and the third marker is CRP.

In an alternative embodiment, the first marker is cMyBPC, the second marker is FGF23 and the third marker is ANG2.

In an alternative embodiment, the first marker is cMyBPC, the second marker is NTproBNP and the third marker is CHOL.

In an alternative embodiment, the first marker is cMyBPC, the second marker is NTproBNP and the third marker is ANG2.

In an alternative embodiment, the first marker is cMyBPC, the second marker is ANG2 and the third marker is APOAT.

In an alternative embodiment, the first marker is cMyBPC, the second marker is ANG2 and the third marker is LDL.

Definitions of biomarkers

The first biomarker in accordance with the present invention is cMyBPC (cardiac Myosin binding protein C). Myosin-binding protein C is a myosin-associated protein found in the cross-bridge-bearing zone (C region) of A bands in striated muscle.

In accordance with the present invention, the amount of the cardiac isoform, i.e. cMYBPC (cardiac Myosin binding protein C) shall be determined (also referred to as MYBPC3, CMD1MM, CMH4, FHC, LVNC10, MYBP-C, Myosin binding protein C, cardiac, cMyBP-C, myosin binding protein C3). cMyBPC is produced in heart muscle. It is encoded by the MYBPC3 gene. Further information on human cMYBPC can be found in the UniProtKB database under accession number Q14896 (MYPC3_HUMAN).

BMP10-type peptides are well known in the art. Preferred BMP10-type peptide are e.g. disclosed in Susan-Resiga et al. (J Biol Chem. 2011 Jul 1;286(26):22785-94) (see e.g. Figure 3A of Susan-Resiga et al., or US 2012/0213782).

The BMP-type peptide is preferably, NT-proBMP10, proBMP10 or BMP, more preferably, NT-proBMP10 or proBMP 10, and most preferably NT-proBMP10.

In an embodiment, the BMP10-type peptide is unprocessed preproBMP10. In another embodiment, the BMP10-type peptide is the propeptide proBMP10. This marker comprises the N-terminal prosegement and BMP10. In another embodiment, the BMP10-type peptide is the N-terminal prosegment of BMP10 (N-terminal proBMP10). In another embodiment, the BMP10-type peptide is BMP10.

In an embodiment, the BMP10-type peptide is part of a homo- or heterodimeric complex. Human preproBMP10 (i.e. unprocessed preproBMP10) has a length of 424 amino acids. The amino acid sequence of human preproBMP10 is e.g. shown in SEQ ID NO: 1 of WO 2020/035605 A1 or in Fig. 3 of US 2012/0213782. SEQ ID NO: 1 of WO 2020/035605 A1 is the same sequence as SEQ ID NO: 2 in the sequence listing of the present application. Further, the amino acid sequence of preproBMP10 can be assessed via Uniprot (see sequence under accession number 095393-1). Human preproBMP10 comprises a short signal peptide (amino acids 1 to 21) which is enzymatically cleaved off to release proBMP10. Accordingly, human proBMP10 comprises amino acids 22 to 424 of human preproBMP10 (i.e. of the polypeptide having a sequence shown in SEQ ID NO 1 of WO 2020/035605 A1). Human proBMP10 is further cleaved into an N-terminal prosegment of BMP10 and (non-glycosylated) BMP10 which is the active form. The N-terminal prosegment of BMP10 comprises amino acids 22 to 316 of the polypeptide having a sequence shown in SEQ ID NO 1 of WO 2020/035605 A1 (i.e. of human preproBMP10). BMP10 comprises amino acids 317 to 424 of the polypeptide having a sequence shown in SEQ ID NO 1 of WO 2020/035605 A1.

The preferred BMP10-type peptides are BMP10 and N-terminal proBMP10. After cleavage of proBMP10, BMP10 and N-terminal proBMP10 remain in structural proximity forming homo- or hetero-dimers of BMP10 or in combination with other BMP-family proteins (Yadin et al., CYTOGFR 2016, 27 (2016) 13-34). The dimerization occurs by formation of Cys-Cys bridge or strong adhesion in the C-terminal peptides of both binding partners. Thus, an architecture consisting of two subunits is formed.

Since proBMP10 is cleaved into BMP10 and the N-terminal prosegment in equimolar proportions, the amount of BMP10 reflects the amount of the N-terminal prosegment. Thus, the amount of BMP10 can be determined by determining the amount of the N-terminal prosegment and *vice versa.*

Preferably, the amount of the BMP10-type peptide is determined by using one or more antibodies (or antigen-binding fragments thereof) which specifically bind to the BMP10-type peptide.

For example, one or more antibodies which specifically bind to the N-terminal prosegment of BMP10 could be used. Since such antibodies (or fragments) would also bind to proBMP10 and preproBMP10, the sum of the amounts of the N-terminal prosegment of BMP10, proBMP10 and preproBMP10 is determined in step a) of the methods of the present invention. Accordingly, the expression "determining the amount of the N-terminal prosegment of BMP10" also shall mean "determining the sum of the amounts of the N-terminal prosegment of BMP10, proBMP10 and preproBMP10".

Structural prediction based on findings from other BMP-type proteins as e.g. BMP9 show that BMP10 remains in a complex with proBMP10, thus detection of N-term prosegement also reflects the amount of BMP10.

For example, one or more antibodies which specifically bind to BMP10 could be used. Since such antibodies (or fragments) would also bind to proBMP10 and preproBMP10, the sum of the amounts of the BMP10, proBMP10 and preproBMP10 is determined in step a) of the methods of the present invention. Accordingly, the expression "determining the amount of BMP10" also shall mean "determining the sum of the amounts of BMP10, proBMP10 and preproBMP10".

Structural prediction based on findings from other BMP-type proteins as e.g. BMP9 show that BMP10 remains in a complex with proBMP10, thus detection of BMP10 also reflects the amount of N-terminal prosegment.

Further, it is envisaged to determine the sum of the amounts of all four BMP10-type peptides as referred to above, i.e. of BMP10, the N-terminal prosegment of BMP10, proBMP10 and preproBMP10.

Accordingly, the following amounts of BMP10-type peptides can be determined in accordance with the present invention:

- the amount of BMP10
- the amount of the N-terminal prosegment of BMP10
- the amount of proBMP10
- the amount of preproBMP10
- the sum of the amounts of BMP10, proBMP10 and preproBMP10
- the sum of the amounts of the N-terminal prosegment of BMP10, proBMP10 and preproBMP10, or
- the sum of the amounts of BMP10, the N-terminal prosegment of BMP10, proBMP10 and preproBMP10

In an embodiment the amount of the BMP10-type peptide is determined as described in WO 2020/035605 A1. In another embodiment, the amount of the BMP10 type-peptide is determined as described in WO 2021/165465 A1. In the Examples section of the present application, the amount of the BMP10-type peptide was determined by using antibodies binding to NT-proBMP10.

In another embodiment, the second biomarker is FGF23. The biomarker fibroblast growth factor-23 (abbreviated "FGF-23") is well known in the art. FGF-23 is a key player in the regulation of calcium-phosphate and vitamin D metabolism and has a causal role in the pathogenesis of LV hypertrophy, a major determinant of cardiovascular events, and kidney disease. Preferably, FGF-23 is human FGF-23. The sequence of human FGF-23 is well known in the art, e.g. the amino sequence sequence can be assessed via GenBank accession number NM _020638_ 1 GI:10190673. Moreover, the sequence is also disclosed in Shimada et al., 2001, PNAS, vol. 98(11) page 6500 to 6505.

The Brain Natriuretic Peptid type peptide (herein also referred to as BNP-type peptide) is preferably selected from the group consisting of pre-proBNP, proBNP, NT-proBNP, and BNP. The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP). Preferably, brain natriuretic peptides according to the present invention are NT-proBNP, BNP, and variants thereof. BNP is the active hormone and has a shorter half-life than its respective inactive counterpart NT-proBNP. Preferably, the Brain Natriuretic Peptid-type peptide is BNP (Brain natriuretic peptide), and more preferably NT-proBNP (N-terminal of the prohormone brain natriuretic peptide).

In an embodiment, the BNP-type peptide is BNP.

In another embodiment, the BNP-type peptide is NT-proBNP.

NT-proBNP can be "total NT-proBNP" (herein also referred to as tNT-proBNP) or "unglycosylated NT-proBNP", such as NT-proBNP which is unglycosylated at position S44.

The sequence of human NT-proBNP is well known in the art and has been described already in detail in the prior art, e.g., WO 02/089657, WO 02/083913, Bonow 1996, New Insights into the cardiac natriuretic peptides. Circulation 93: 1946-1950. Preferably, human NT-proBNP has an amino acid sequence as shown in SEQ ID NO: 1.

NT-proBNP as well as its precursor proBNP can be O-glycosylated. An overview is on the O-Glycosylation of NT-proBNP and proBNP is, e.g., provided in by Schellenberger et al. and Halflinger et al. (Schellenberger et al., Arch Biochem Biophys 2006; 451; Halfinger et al., Clinical Chemistry 63:1, 359-368 (2017)).

The term "O-linked glycosylation" is well known in the art (herein also referred to as "glycosylation"). O-linked glycosylation is the attachment of a sugar molecule, i.e. a carbohydrate to serine or threonine residues. It is a post-translational modification that occurs after the protein has been synthesized. How NT-proBNP and proBNP are glycosylated is, for example, described in Schellenberger et al. and Halflinger et al. (cited above).

An overview on O-Glycosylation sites of NT-proBNP is provided herein below. The following sequence is the human NT-proBNP sequence (SEQ ID NO: 1 which serves as a reference sequence here). The O-Glycosylation sites are underlined:

```
His Pro Leu Gly Ser Pro Gly Ser Ala Ser Asp Leu Glu Thr Ser Gly
1            5               10              15
Leu Gln Glu Gln Arg Asn His Leu Gln Gly Lys Leu Ser Glu Leu Gln
            20              25              30
Val Glu Gln Thr Ser Leu Glu Pro Leu Gln Glu Ser Pro Arg Pro Thr
        35              40              45
Gly Val Trp Lys Ser Arg Glu Val Ala Thr Glu Gly Ile Arg Gly His
    50              55              60
Arg Lys Met Val Leu Tyr Thr Leu Arg Ala Pro Arg (SEQ ID NO: 1)
65              70              75
```

Thus, human NT-proBNP (as shown in SEQ ID NO: 1) comprises at least the following glycosylation sites: T36, S37, S44, T48, S53, T58 and T71.

Preferably, the term "unglycosylated NT-proBNP" as used herein, refers to NT-proBNP which is not glycosylated (i.e. which is not O-glycosylated) at one or more positions selected from group consisting of T36, S37, S44, T48, S53, T58 and T71 of human NT-proBNP. Thus, at least one of the following amino acid residues of human NT-proBNP is not glycosylated, i.e. does not comprise an O-glycosylation: T36, S37, S44, T48, S53, T58 and T71.

In some embodiments, the term "unglycosylated NT-proBNP", refers to NTproBNP in which at least two of the aforementioned amino acid residues (i.e. of T36, S37, S44, T48, S53, T58 and T71) are not glycosylated. In some embodiments, the term "unglycosylated NT-proBNP", refers to NTproBNP in which at least three of the aforementioned amino acid residues are not glycosylated. In some embodiments, the term "unglycosylated NT-proBNP", refers to NTproBNP in which at least three, at least four, at least five, at least six, or all of the aforementioned amino acid residues are not glycosylated. In some embodiments, the term "unglycosylated NT-proBNP", refers to NTproBNP in which at least the serine residue at position 44 (i.e. S44) is not glycosylated. Thus, unglycosylated NT-proBNP may be unglycosylated at position S44 (i.e. Ser 44).

In a preferred embodiment, determining of the amount of unglycosylated NT-proBNP in a sample from the subject comprises contacting the sample with an antibody, or antigen-binding fragment thereof, which specifically detects unglycosylated NT-proBNP. Preferably, the antibody, or antigen-binding fragment thereof, which specifically detects unglycosylated NT-proBNP specifically binds to an epitope of NT-proBNP which epitope comprises a glycosylation site, but which is not glycosylated at the glycosylation site. The formed complex between the antibody (or fragment) and the biomarker shall be proportional to the amount of the unglycosylated NT-proBNP.

In some embodiments, the antibody, or antigen-binding fragment thereof, which specifically detects unglycosylated NT-proBNP specifically binds to an epitope of NT-proBNP which epitope comprises the T36 amino acid residue, wherein said T36 amino acid residue is not glycosylated. Preferably, said antibody or fragment essentially does not bind NT-proBNP comprising a glycosylated T36 amino acid residue.

In some embodiments, said antibody, or fragment thereof, specifically binds to an epitope of NT-proBNP which epitope comprises S37 amino acid residue, wherein said S37 amino acid residue is not glycosylated. Preferably, said antibody or fragment essentially does not bind NT-proBNP comprising a glycosylated S37 amino acid residue.

In some embodiments, said antibody, or fragment thereof, specifically binds to an epitope of NT-proBNP which epitope comprises the S44 amino acid residue, wherein said S44 amino acid residue is not glycosylated. Preferably, said antibody or fragment essentially does not bind NT-proBNP comprising a glycosylated S44 amino acid residue. In some embodiments, the epitope of said antibody, or antigen-binding fragment thereof, comprises amino acid residues 42 to 46 of human NT-proBNP (as shown in SEQ ID NO: 1).

In a preferred embodiment, the antibody which specifically detects unglycosylated NT-proBNP is the monoclonal antibody MAB 1.21.3 as disclosed in WO2004099253A1, or an antibody, which comprises the six CDRs of said antibody. Further, it is envisaged to use an antigen-binding fragment of said antibody.

In some embodiments, said antibody, or fragment thereof, specifically binds to an epitope of NT-proBNP which epitope comprises the T48 amino acid residue, wherein said T48 amino acid residue is not glycosylated. Preferably, said antibody or fragment essentially does not bind NT-proBNP comprising a glycosylated T48 amino acid residue.

In some embodiments, said antibody, or fragment thereof, specifically binds to an epitope of NT-proBNP which epitope comprises the S53 amino acid residue, wherein said S53 amino acid residue is not glycosylated. Preferably, said antibody or fragment essentially does not bind NT-proBNP comprising a glycosylated S53 amino acid residue.

In some embodiments, said antibody, or fragment thereof, specifically binds to an epitope of NT-proBNP which epitope comprises the T58 amino acid residue, wherein said T58 amino acid residue is not glycosylated. Preferably, said antibody or fragment essentially does not bind NT-proBNP comprising a glycosylated T58 amino acid residue.

In some embodiments, said antibody, or fragment thereof, specifically binds to an epitope of NT-proBNP which epitope comprises the T71 amino acid residue, wherein said T71 amino acid residue is not glycosylated. Preferably, said antibody or fragment essentially does not bind NT-proBNP comprising a glycosylated T71 amino acid residue.

Preferably, the term "glycosylated NT-proBNP" as used herein, preferably, refers to NT-proBNP which glycosylated (i.e. O-glycosylated) at one or more positions (i.e. amino acid residues) selected from group consisting of T36, S37, S44, T48, S53, T58 and T71 of human NT-proBNP. Thus, at least one of the following amino acid residues of human NT-proBNP is glycosylated, i.e. comprises an O-glycosylation: T36, S37, S44, T48, S53, T58 and T71.

In some embodiments, the term "glycosylated NT-proBNP", refers to NTproBNP in which at least two of the aforementioned amino acid residues (i.e. of T36, S37, S44, T48, S53, T58 and T71) are glycosylated. In some embodiments, the term "glycosylated NT-proBNP", refers to NTproBNP in which at least three of the aforementioned amino acid residues are glycosylated. In some embodiments, the term "glycosylated NT-proBNP", refers to NTproBNP in which at least three, at least four, at least five, at least six, or all of the aforementioned amino acid residues are glycosylated. In some embodiments, the term "glycosylated NT-proBNP", refers to NTproBNP in which at least the serine residue at position 44 (i.e. S44) is glycosylated.

The "total amount of NT-proBNP" is preferably the amount of glycosylated and unglycosylated NT-proBNP. The term, thus, refers to the sum of the amount of glycosylated NT-proBNP and unglycosylated NT-proBNP.

Preferably, the determination of the amount of total NT-proBNP comprises contacting the sample with an antibody, or antigen-binding fragment thereof, which specifically detects total NT-proBNP. More preferably, said antibody, or antigen-binding fragment thereof, which specifically detects total NT-proBNP binds to a region of human NT-proBNP which can not be glycosylated. Accordingly, said antibody (or fragment thereof) shall specifically bind to a region of NT-proBNP, in particular of human NT-proBNP, which does not carry a glycosylation site, i.e. an O-glycosylation site. The formed complex between the antibody (or fragment) and the biomarker shall be proportional to the amount of the total NT-proBNP.

In particular, said antibody (or fragment thereof) shall specifically bind to a region of NT-proBNP which does not carry the T36, S37, S44, T48, S53, T58 or T71 glycosylation site (of human NT-proBNP). For example, the first 35 amino acid residues, i.e. the N-terminal amino acid residues 1 to 35 are known to carry no O-glycosylation sites. Preferably, the antibody, or antigen-binding fragment thereof, which specifically detects total NT-proBNP binds to an epitope present within the first 35 amino acids of NT-proBNP, more preferably, it binds to an epitope present within the first 20 amino acids of NT-proBNP, most preferably, it binds to an epitope present within amino acids residues 10 to 20 of human NT-proBNP. In a preferred embodiment, the epitope of said antibody, or antigen-binding fragment thereof, comprises amino acid residues 13 to 16 of human NT-proBNP. The sequence of human NT-proBNP is shown above (see SEQ ID NO: 1).

In a preferred embodiment, the antibody which specifically detects total NT-proBNP is the monoclonal antibody MAB 17.3.1 as disclosed in WO2004099253A1, or an antibody, which comprises the six CDRs of said antibody. Further, it is envisaged to use an antigen-binding fragment of said antibody.

The biomarker Angiopoietin-2 (abbreviated "ANG2", frequently also referred to as ANGPT2) is well known in the art. It is a naturally occurring antagonist for both Ang-1 and TIE2 (see e.g. Maisonpierre et al., Science 277 (1997) 55-60). The protein can induce tyrosine phosphorylation of TEK/TIE2 in the absence of ANG-1. In the absence of angiogenic inducers, such as VEGF, ANG2-mediated loosening of cell-matrix contacts may induce endothelial cell apoptosis with consequent vascular regression. In concert with VEGF, it may facilitate endothelial cell migration and proliferation, thus serving as a permissive angiogenic signal. The sequence of human Angiopoietin is well known in the art. Uniprot lists three isoforms of Angiopoietin-2: Isoform 1 (Uniprot identifier: 015123-1), Isoform 2 (identifier: 015123-2) and Isoform 3 (O15123-3). In a preferred embodiment, the total amount of Angiopoietin-2 is determined. The total amount is preferably the sum of the amounts of complexed and free Angiopoietin-2.

The term "Growth-Differentiation Factor-15" or "GDF-15" relates to a polypeptide being a member of the transforming growth factor (TGF) cytokine superfamily. The terms polypeptide, peptide and protein are used interchangeable throughout this specification. GDF-15 was originally cloned as macrophage-inhibitory cytokine 1 and later also identified as placental transforming growth factor-15, placental bone morphogenetic protein, non-steroidal anti-inflammatory drug-activated gene 1, and prostate-derived factor (Bootcov loc cit; Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Amino acid sequences for GDF-15 are disclosed in WO99/06445, WO00/70051, WO2005/113585, Bottner 1999, Gene 237: 105-111, Bootcov loc. cit, Tan loc. cit., Baek 2001, Mol Pharmacol 59: 901-908, Hromas loc cit, Paralkar loc cit, Morrish 1996, Placenta 17:431-441.

CRP (C-reactive protein) is an acute phase protein that was discovered more than 75 years ago to be a blood protein that binds to the C-polysaccharide of pneumococci. CRP is known as a reactive inflammatory marker and is produced by a distal organ (i.e. the liver) in response or reaction to chemokines or interleukins originating from the primary lesion site. CRP is known to consist of five single subunits, which are non-covalently linked and assembled as a cyclic pentamer with a molecular weight of approximately 110-140 kDa. Preferably, CRP as used herein relates to human CRP. The sequence of human CRP is well known and disclosed, e.g., by Woo et al. (J. Biol. Chem. 1985. 260 (24), 13384-13388). The level of CRP is usually low in normal individuals but can rise 100- to 200-fold or higher due to inflammation, infection or injury (Yeh (2004) Circulation. 2004; 109:11-11-11-14). It is known that CRP is an independent factor for the prediction of a cardiovascular risk. CRP can be determined by immunoassays, e.g., ELISAs, that are well known in the art and are commercially available. In a preferred embodiment, CRP is hsCRP (high sensitivity CRP).

The biomarker endothelial cell specific molecule 1 (abbreviated ESM-1) is well known in the art. The biomarker is frequently also referred to as endocan. ESM-1 is a secreted protein which is mainly expressed in the endothelial cells in human lung and kidney tissues. Public domain data suggest expression also in thyroid, lung and kidney, but also in heart tissue, see. e.g. the entry for ESM-1 in the Protein Atlas database (Uhlén M. et al., Science 2015;347(6220): 1260419). The expression of this gene is regulated by cytokines. ESM-1 is a proteoglycan composed of a 20 kDa mature polypeptide and a 30 kDa O-linked glycan chain (Bechard D et al., J Biol Chem 2001;276(51):48341-48349). In a preferred embodiment of the present invention, the amount of the human ESM-1 polypeptide is determined in a sample from the subject. The sequence of the human ESM-1 polypeptide is well known in the art (see e.g. Lassale P. et al., J. Biol. Chem. 1996;271:20458-20464 and can be e.g. assessed via Uniprot database, see entry Q9NQ30 (ESM1 _HUMAN). Two isoforms of ESM-1 are produced by alternative splicing, isoform 1 (having the Uniprot identifier Q9NQ30-1) and isoform 2 (having the Uniprot identifier Q9NQ30-2). Isoform 1 has length of 184 amino acids. In isoform 2, amino acids 101 to 150 of isoform 1 are missing. Amino acids 1 to 19 form the signal peptide (which might be cleaved off).

In a preferred embodiment, the amount of isoform 1 of the ESM-1 polypeptide is determined, i.e. isoform 1 having a sequence as shown under UniProt accession number Q9NQ30-1.

In another preferred embodiment, the amount of isoform 2 of the ESM-1 polypeptide is determined, i.e. isoform 2 having a sequence as shown under UniProt accession number Q9NQ30-2.

In another preferred embodiment, the amount of isoform-1 and isoform 2 of the ESM-1 polypeptide is determined, i.e. total ESM-1.

In accordance with the present invention, the amount of one or more lipid biomarkers may be determined.

The lipid biomarker marker as referred to herein is preferably a lipid biomarker selected from the group consisting of Cholesterol (herein also referred to as "CHOL"), TAG (Triglycerides, herein also referred to as "TRIGLY"), LDL (Low-density Lipoprotein), HDL (High-density Lipoprotein), and Apolipoprotein A-1 (herein also referred to as "APOAT"). Thus, one or more markers from the aforementioned group of markers can be determined.

In an embodiment, the lipid biomarker is Cholesterol. Cholesterol is a steroid with a secondary hydroxyl group in the C3 position. It is synthesized in many types of tissue, but particularly in the liver and intestinal wall. Approximately three quarters of cholesterol is newly synthesized and a quarter originates from dietary intake. Cholesterol assays are used for screening for atherosclerotic risk and in the diagnosis and treatment of disorders involving elevated cholesterol levels as well as lipid and lipoprotein metabolic disorders.

In another embodiment, the lipid biomarker is Apolipoprotein A-1. Apolipoproteins are the protein constituents of the lipoproteins. The lipoproteins are classified according to their ultracentrifugal flotation density. Apolipoprotein A-1 is the major protein constituent of high-density lipoproteins (HDL). Apolipoprotein A-1 activates the enzyme lecithin-cholesterol-acyltransferase (LCAT), which catalyzes the esterification of cholesterol, thereby enhancing the lipid-carrying capacity of the lipoproteins. Further information on is Apolipoprotein A-1 under UniProt-Accession-Number UniProtKB - P02647 (APOA1_HUMAN).

In yet another embodiment, the lipid biomarker are triglycerides. Triglycerides are esters of the trihydric alcohol glycerol with three long-chain fatty acids. They are partly synthesized in the liver and partly ingested in food. The determination of triglycerides is utilized in the diagnosis and treatment of patients having diabetes mellitus, nephrosis, liver obstruction, lipid metabolism disorders and numerous other endocrine diseases.

In yet another embodiment, the lipid biomarker is LDL. Thus, the LDL content is determined. Low Density Lipoproteins (LDL) play a key role in causing and influencing the progression of atherosclerosis and, in particular, coronary sclerosis. LDL is one of the five major groups of lipoprotein which transport all fat molecules around the body in the extracellular water. Blood tests commonly report LDL-Cholesterol: the amount of cholesterol which is estimated to be contained with LDL particles, on average, by using the Friedewald equation. Furthermore, the LDL content can assessed via the cholesterol content in LDL.

In yet another embodiment, the lipid biomarker is HDL. HDL are synthesized by the intestines and the liver. High density lipoprotein (HDL) is responsible for the reverse transport of cholesterol from the peripheral cells to the liver. Monitoring of HDL-cholesterol in serum or plasma is of clinical relevance as the HDL-cholesterol concentration is important in the assessment of atherosclerotic risk.

The term "determining" as used herein refers to qualitative and quantitative determination of the biomarkers referred to in accordance with the present invention, i.e. the term encompasses the determination of the presence or absence or the determination of the absolute or relative amount of said biomarkers.

The term "amount" as used herein refers to the absolute amount of a compound referred to herein, the relative amount or concentration of the said compound as well as any value or parameter which correlates thereto or can be derived therefrom. Such values or parameters comprise intensity signal values from all specific physical or chemical properties obtained from the said compounds by direct measurements, e.g., intensity values in mass spectra or NMR spectra. Moreover, encompassed are all values or parameters which are obtained by indirect measurements specified elsewhere in this description, e.g., response levels determined from biological read out systems in response to the compounds or intensity signals obtained from specifically bound ligands. It is to be understood that values correlating to the aforementioned amounts or parameters can also be obtained by all standard mathematical operations.

Determining the amount in the method of the present invention may be carried out by any technique which allows for detecting the presence or absence or the amount of said second molecule upon its release from the first molecule. Suitable techniques depend on the molecular nature and the properties of the biomarkers and are discussed elsewhere herein in more detail.

Typically, the amount of a biomarker as referred to in accordance with the present invention can be determined by immunoassays using sandwich, competition, or other assay formats, in particular, if the biomarker is a protein biomarker (such as a BNP-type peptide, a BMP10-type peptide, GDF-15, ANG2, CRP, or ESM1, APOAT). Said assays will develop a signal which is indicative for the presence or absence or the amount of a biomarker. Further suitable methods comprise measuring a physical or chemical property specific for the biomarker such as its precise molecular mass or NMR spectrum. Said methods comprise, preferably, biosensors, optical devices coupled to immunoassays, biochips, analytical devices such as mass-spectrometers, NMR-analysers, surface plasmon resonance measurement equipment or chromatography devices. Further, methods include micro-plate ELISA-based methods, fully-automated or robotic immunoassays (available, e.g., from Roche). Suitable measurement methods according to the present invention may also include precipitation (particularly immunoprecipitation), electrochemiluminescence (electro-generated chemiluminescence), RIA (radioimmunoassay), ELISA (enzyme-linked immunosorbent assay), electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods are known in the art such as gel electrophoresis, 2D gel electrophoresis, SDS polyacrylamid gel electrophoresis (SDS-PAGE) or Western Blotting. More typically, techniques particular envisaged for determining the biomarkers referred to herein are described in the accompanying Examples, below.

The biomarkers to be determined in accordance with the present invention are well-known in the art. Moreover, methods for the determination of the amount of the biomarkers are known. For example, the biomarkers can be measured as described in the Examples section (see Example 1).

Some of the biomarkers are the lipid biomarkers HDL, LDL, Cholesterol and triglycerides. The amount of these biomarkers can be determined e.g. enzymatically.

The enzymatic determination of the amount of (total) triglycerides, preferably, comprises the steps of:

a) contacting the sample with lipase under conditions and for a time sufficient to allow conversion into glycerol and free fatty acids;

b) contacting the sample comprising the glycerol with glycerokinase under conditions and for a time sufficient to allow conversion into glycerol-3-phosphate;

c) contacting the sample comprising the glycerol-3-phosphate with glycerophosphate oxidase under conditions and for a time sufficient to allow conversion into dihydroxyacetone phosphate and $H_2O_2$; and

d) enzymatically or chemically determining the amount of generated $H_2O_2$, thereby determining the amount of triglycerides,

Thus, detection agents for triglycerides are the enzymes: glycerokinase, glycerophosphate oxidase and glycerophosphate oxidase. Preferably, these enzymes are used in the combination.

The amount of cholesterol may be assessed via the amount of cholesterol esters in a sample. The enzymatic determination of the amount of cholesterol esters, preferably, comprises the steps of:

a) contacting the sample with cholesterol esterase under conditions and for a time sufficient to allow conversion into cholesterol;

b) contacting the sample comprising the cholesterol with cholesterol oxidase under conditions and for a time sufficient to allow generation of $H_2O_2$; and

c) enzymatically or chemically determining the amount of generated $H_2O_2$, thereby determining the amount of Cholesterol.

Thus, detection agents for cholesterol esters are the enzymes: cholesterol esterase and cholesterol oxidase. Preferably, these enzymes are used in the combination.

As set forth above, the amount of LDL may be assessed by determining the LDL cholesterol and cholesterol esters. The enzymatic determination of the amount of LDL cholesterol and cholesterol esters, preferably, comprises the steps of:

a) contacting the sample in the presence with of a non-ionic detergent which selectively solubilizes LDL with cholesterol esterase under conditions and for a time sufficient to allow conversion into cholesterol;

b) contacting the sample comprising the cholesterol with cholesterol oxidase under conditions and for a time sufficient to allow generation of $H_2O_2$; and

c) enzymatically or chemically determining the amount of generated $H_2O_2$, thereby determining the amount of LDL cholesterol and cholesterol esters, and thus of LDL.

Thus, detection agents for HDL are the enzymes: cholesterol esterase and cholesterol oxidase. Preferably, these enzymes are used in the combination.

As set forth above, the amount of HDL may be assessed by determining the HDL cholesterol and cholesterol esters, i.e. the amount cholesterol and cholesterol esters present in HDL The enzymatic determination of the amount of LDL cholesterol and cholesterol esters, preferably, comprises the steps of:

a) contacting the sample in the presence with of a non-ionic detergent which selectively solubilizes LDL with cholesterol esterase under conditions and for a time sufficient to allow conversion into cholesterol;

b) contacting the sample comprising the cholesterol with cholesterol oxidase under conditions and for a time sufficient to allow generation of $H_2O_2$; and

c) enzymatically or chemically determining the amount of generated $H_2O_2$, thereby determining the amount of LDL cholesterol and cholesterol esters, and thus of LDL.

Thus, detection agents for LDL are the enzymes: cholesterol esterase and cholesterol oxidase. Preferably, these enzymes are used in the combination.

The amount of $H_2O_2$ can be e.g. enzymatically determined by converted in a further step (step e or d) in which the sample is contacted with a peroxidase (such as a horseradish peroxidase) and a chromogenic substrate. The substrate is typically oxidized by the peroxidase using $H_2O_2$ as the oxidizing agent. The catalyzed reaction in the presence of $H_2O_2$, peroxidase, and the substrate typically results in a characteristic change that is detectable by spectrophotometric methods. E.g., the peroxidase catalyzes the conversion of chromogenic substrates into colored products, and produces light when acting on chemiluminescent sub-strates. For example, DAOS (N-ethyl-N-(2-hydroxy-3-sulfopropyl)-3,5-dimethoxyaniline) plus 4-aminoantipyrine in the presence of $H_2O_2$ and peroxidase results in the oxidative coupling of DAOS and 4-aminoantipyrine to form a blue chromogen. This chromogen can be e.g. detected by measuring the absorbance of light at about 590 nm. Alternatively, 10-acetyl-3,7-dihydroxyphenoxazine can be used as substrate for peroxidase that enables detection of $H_2O_2$. This non-fluorescent reagent reacts with $H_2O_2$ to produce resorufin, a fluorescent compound. In another alternative 4-aminophenazon plus 4-chlorphenol in the presence of $H_2O_2$ and

peroxidase result in the formation of 4-(p-benzochinon-monoimino)-phenazon, a red colored product, which can be determined by photometric means.

In addition to the first, second and third marker determined according to the claims, the invention may encompass the determination of additional markers.

The term "reference" as used herein, preferably, refers to an amount, or score which allows for allocation of a subject into either the group of subjects suffering from a disease or condition, for example type 2 myocardial infarction, or from type 1 myocardial infarction (MI). Such a reference can be a threshold (or cutoff) amount or score which separates these groups from each other. Accordingly, the reference shall be an amount or score which allows for allocation of a subject into a group of subjects suffering from a type 1 or 2 myocardial infarction. For example, the reference shall be an amount or score which allows for allocation of a subject into a group of subjects suffering from type 2 myocardial infarction, or not suffering from type 2 myocardial infarction.

In an embodiment, the reference is a reference amount. In another embodiment the reference is a reference score

The term "at least one subject" refers to one subject or more than one subject, such as at least 10, 50, 100, 200, or 1000 subjects.

Reference amounts can, in principle, be calculated for a cohort of subjects based on the average or mean values for a given parameter such as biomarker amount by applying standard statistically methods. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity/specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis of a disease of condition with a proper balance of sensitivity and specificity, respectively. For example, the reference to be used for the aforementioned method of the present invention, i.e. a threshold which allows to differentiate between type 1 and type 2 myocardial infarction can be generated, usually, by establishing a ROC for said cohort as described above and deriving a threshold amount therefrom. Dependent on a desired sensitivity and specificity for a diagnostic method, the ROC plot allows deriving suitable thresholds.

It is to be understood that references shall allow for the assessment as referred to herein. For example, it shall allow from differentiating between type 1 and type 2 myocardial infarction, or for making suitable treatment decisions.

Step c) of the method of the present invention comprises comparing the amounts of the biomarkers (i.e. the first biomarker, the second biomarker and optionally the third biomarker) to references for said biomarkers and/or calculating a score for assessing myocardial infarction based on the amounts of the biomarkers.

Thus, the amount of the first biomarker, the second biomarker and optionally the third biomarker, respectively, may be compared to a reference for the first biomarker, a reference for the second biomarker and optionally a reference for the third biomarker.

Alternatively, a score may be calculated based on the amounts of the biomarkers, i.e. based on the amounts of the first biomarker, the second biomarker and, optionally, the third biomarker. Said score shall allow for assessing myocardial infarction, such as for differentiating between type 1 and type 2 myocardial infarction. Optionally, said score may be compared to a suitable reference score.

The term "comparing" as used herein encompasses comparing the determined amount for a biomarker as referred to herein to a reference (or of a calculated score to a reference score) It is to be understood that comparing as used herein refers to any kind of comparison made between the value for the amount with the reference. However, it is to be understood that, preferably, identical types of values are compared with each other, e.g., if an absolute amount is determined and to be compared in the method of the invention, the reference shall also be an absolute amount, if a relative amount is determined and to be compared in the method of the invention, the reference shall also be a relative amount, etc.. Alternatively, the term "comparing" as used herein encompasses comparing a calculated score with a suitable reference core. The comparison may be carried out manually or computer assisted. The value of the amount or the score and the reference can be, e.g., compared to each other and the said comparison can be automatically carried out by a computer program executing an algorithm for the comparison. The computer program carrying out the said evaluation will provide the desired assessment in a suitable output format.

As set forth above, it is also envisaged to combine multiple biomarkers into a single score based on the amounts of the first and second biomarker, or the first, second or third biomarker, i.e. a single score, and to compare this score to a reference score. Thus, a score is calculated. Preferably, the score is based on the amounts of the first and second biomarker in the sample from the test subject, and, if the amount of the third biomarker is determined, on the amounts of first, second and third biomarker in the sample from the test subject. Although the score shall be based on the amounts of the aforementioned biomarkers, further biomarkers or further patient specific parameters may contribute to the score.

The calculated score combines information on the amounts of the at least two biomarkers. Moreover, in the score, the biomarkers are, preferably, weighted in accordance with their contribution to the establishment of the assessment. Thus, the values for the individual markers are weighted and the weighted values are used for calculating the score. Suitable coefficients (weights) can be determined by the skilled person without further ado. A score can also be calculated from a decision tree or a set (ensemble) of decision trees that has been trained on at least two biomarkers. Based on the combination of biomarkers applied in the method of the invention, the weight of an individual biomarker as well as the structure of decision trees may be different.

The score can be regarded as a classifier parameter for assessing a subject as set forth herein. In particular, it enables the person who provides the assessment based on a single score. The reference score is preferably a value, in particular a cut-off value which allows for assessing myocardial infarction as set forth herein. Preferably, the reference is a single value. Thus, the person does not have to interpret the entire information on the amounts of the individual biomarkers. Using a scoring system as described herein, advantageously, values of different dimensions or units for the biomarkers may be used since the values will be mathematically transformed into the score. Accordingly, e.g. values for absolute concentrations may be combined in a score with peak area ratios. The reference score to be applied may be elected based on the desired sensitivity or the desired specificity. How to elect a suitable reference score is well known in the art.

The score shall allow for the assessment for myocardial infarction, such as for the differentiation between type 2 and type 1 myocardial infarction. How to calculate a score, is well known in the art. An example is provided in the Examples section, see Example 2, *Example of use of cMyBPC and BMP 10 combination*). A suitable diagnostic algorithm can be set up by the skilled person as well. For example, a calculated score which is larger than the reference score is indicative for a subject who suffers from type 2 MI, whereas a calculated score which is lower than the reference score is indicative for the a subject who suffers from type 1 MI.

For the specific markers as forth herein preferred changes ("directions") are found in following table.

Table A: direction of the biomarkers

| Biomarker | Direction |
|---|---|
| cMyBPC | Elevated in Type 1 MI, decreased in Type 2 MI |
| LDL | Elevated in Type 1 MI, decreased in Type 2 MI |
| TRIGL | Elevated in Type 1 MI, decreased in Type 2 MI |
| CHOL | Elevated in Type 1 MI, decreased in Type 2 MI |
| ANG2 | Elevated in Type 2 MI, decreased in Type 1 MI |
| BMP10-type peptide | Elevated in Type 2 MI, decreased in Type 1 MI |
| ESM1 | Elevated in Type 2 MI, decreased in Type 1 MI |
| FGF23 | Elevated in Type 2 MI, decreased in Type 1 MI |
| tproBNP | Elevated in Type 2 MI, decreased in Type 1 MI |
| proBNP | Elevated in Type 2 MI, decreased in Type 1 MI |
| GDF15 | Elevated in Type 2 MI, decreased in Type 1 MI |
| APOAT | Elevated in Type 2 MI, decreased in Type 1 MI |
| CRPhs | Elevated in Type 2 MI, decreased in Type 1 MI |

(continued)

| Biomarker | Direction |
|---|---|
| HDL | Elevated in Type 2 MI, decreased in Type 1 MI |

Thus, cardiac troponins, cMyBPC, LDL, TRIGL (Triglycerides, TAG), CHOL, ANG2 and BMP10 are increased in patients with Type 1 MI (as compared to the reference) and decreased in patients with Type 2 MI (as compared to the reference). The remaining markers shown in Table A are decreased in patients with Type 1 MI (as compared to the reference) and increased in patients with Type 2 MI (as compared to the reference).

The methods of the present invention may further comprise recommending or initiating a suitable therapeutic measure. Typically, said suitable therapeutic measure depends on the result of the assessment as described herein, i.e. on whether the subject suffers from type 1 myocardial infarction or type 2 myocardial infarction. For example, it can be decided whether a patient is subjected to a treatment that aims to treat type 2 myocardial infarction or to a treatment that aims to treat type 1 myocardial infarction. Patients identified to suffer from type 2 MI are subjected to a treatment that aims to treat type 2 MI. Patients identified to suffer from type 1 MI are subjected to a treatment that aims to treat type 1 MI.

The methods of the present invention may further guide the urgency and timing of the therapy options respecting the various information obtained from the biomarker for the AMI subtypes.

In an embodiment, the therapeutic measure to be recommended or initiated if a patient has been assessed to suffer from type 1 myocardial infarction is emergent therapy with reperfusion strategies to address the atherothrombotic plaque rupture with drugs (antiplatelets, anticoagulations) and invasive revascularization procedures (such as percutaneous coronary intervention [PCI], the placement of drug eluting stents, or coronary artery bypass grafting [CABG])).

In an embodiment, the therapeutic measure to be recommended or initiated if a patient has been assessed to suffer from type 2 myocardial infarction are procedures to improve the myocardial oxygen supply-demand, such as addressing conditions of decreased myocardial oxygen supply (hypotension, anemia, bradyarrhythmia, respiratory failure), and addressing conditions of increased myocardial oxygen demand (tachycardia, tachyarrhythmias, severe hypertension, other comorbidities) with procedures and medications such as beta-blockers, vasodilators, anticoagulants and diuretics. In an embodiment, the therapeutic measure to be recommended or initiated if a patient has been assessed with a high biomarker score can guide on the appropriate timing of interventions (immediate, early, or delayed).

The definitions given herein above, apply *mutatis mutandis* to the following.

The present invention also relates to a computer-implemented method for assessing myocardial infarction in a subject, said method comprising the steps of:

(a) receiving a value for the amount of a first biomarker in a sample of the subject, said first biomarker being cMyBPC;
(b) receiving a value for the amount of a second biomarker in a sample of the subject, said second biomarker being a BMP10-type peptide, FGF23, a BNP-type peptide, GDF15, ANG2, CRP (C-reactive protein), ESM1, or a lipid biomarker, such as Cholesterol or LDL;
(c) comparing the values for the amounts of the biomarkers to references for said biomarkers and/or calculating a score for assessing myocardial infarction based on the amounts of the biomarkers; and
(d) assessing myocardial infarction based on the comparison and/or the calculation made in step (c), wherein the assessment of myocardial infarction is i) the differentiation between type 1 and type 2 myocardial infarction, ii) the diagnosis of type 2 myocardial infarction, or iii) the guidance of myocardial infarction therapy.

The term "computer-implemented" as used herein means that the method is carried out in an automated fashion on a data processing unit which is, typically, comprised in a computer or similar data processing device. The data processing unit shall receive values for the amount of the biomarkers. Such values can be the amounts, relative amounts or any other calculated value reflecting the amount as described elsewhere herein in detail. Accordingly, it is to be understood that the aforementioned method does not require the determination of amounts for the biomarkers but rather uses values for already predetermined amounts.

Typically, in step (b) of said method

(i) if the value for the amount of a BMP10-type peptide is received as the second biomarker, the method may further comprise receiving a value for the amount of CRP or at least one lipid biomarker selected from the group consisting of Cholesterol, TAG (Triglycerides), LDL (Low-density Lipoprotein), HDL (High-density Lipoprotein) and Apolipoprotein A-1 as a third biomarker; or
(ii) if the value for the amount of FGF23 is received as the second biomarker, the method may further comprise receiving a value for the amount of a BMP10-type peptide, a BNP-type peptide, CRP, ANG2, or at least one lipid biomarker selected from the group consisting of Cholesterol, TAG (Triglycerides), LDL and HDL as a third biomarker, or

(iii) if the value for the amount of a BNP-type peptide is received as the second biomarker, the method may further comprise receiving a value for the amount of Cholesterol or ANG2, or

(iv) if the value for the amount of a ANG-2 is received as the second biomarker, the method further comprises receiving a value for the amount of APOAT or LDL as a third biomarker.

The present disclosure (not part of the present invention) contemplates a computer program, computer program product or computer readable storage medium having tangibly embedded said computer program, wherein the computer program comprises instructions which, when run on a data processing device or computer, carry out the method of the present invention as specified above. Specifically, the present disclosure further encompasses:

- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer script, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network,
- a computer program product having program code means, wherein the program code means can be stored or are stored on a storage medium, for performing the method according to one of the embodiments described in this description, if the program code means are executed on a computer or on a computer network,
- a data stream signal, typically encrypted, comprising a data for parameters as defined herein elsewhere, and
- a data stream signal, typically encrypted, comprising the assessment provided by the methods of the present invention.

The present invention relates to a device for assessing myocardial infarction in a subject with myocardial infarction, said device comprising:

an evaluation unit comprising a database with stored references for a first biomarker being cMyBPC and a second biomarker, said second biomarker being a BMP10-type peptide, FGF23, a BNP-type peptide, GDF15, ANG2, CRP (C-reactive protein), ESM1, or a lipid biomarker, such as Cholesterol or LDL, and a data processor comprising instructions for carrying out a comparison of the amount of the first biomarker and the second biomarker to references, preferably, as specified in any one of claims 1 to 7 and for assessing myocardial infarction based on the comparison, said evaluation unit being capable of receiving values for the amounts of the biomarkers determined in a sample of the subject, wherein, optionally, said database comprises a stored reference for a third biomarker, said third biomarker being (i) if a BMP10-type peptide is the second biomarker, CRP, ANG2 or at least one lipid biomarker selected from the group consisting of Cholesterol, TAG (Triglycerides), LDL (Low-density Lipoprotein), HDL (High-density Lipoprotein) and Apolipoprotein A-1; (ii) if FGF23 is the second biomarker, a BMP10-type peptide, a BNP-type peptide, CRP, ANG2, or at least one lipid biomarker selected from the group consisting of Cholesterol, TAG (Triglycerides), LDL and HDL, or (iii) if a BNP-type peptide is the second biomarker, Cholesterol or ANG2, or (iv) if ANG2 is the second biomarker, APOAT or LDL, wherein the assessment of myocardial infarction is i) the differentiation between type 1 and type 2 myocardial infarction, ii) the diagnosis of type 2 myocardial infarction, or iii) the guidance of myocardial infarction therapy.

The term "device" as used herein relates to a system comprising the aforementioned units operatively linked to each other as to allow the determination of the amounts of biomarkers and evaluation thereof according to the method of the invention such that an assessment can be provided.

The analyzing unit, typically, comprises at least one reaction zone having a biomarker detection agent for the first and second biomarker and, preferably also the third biomarker, in immobilized form on a solid support or carrier which is to be contacted to the sample. Moreover, in the reaction zone, it is possible to apply conditions which allow for the specific binding of the detection agent(s) to the biomarkers comprised in the sample.

The reaction zone may either allow directly for sample application or it may be connected to a loading zone where the sample is applied. In the latter case, the sample can be actively or passively transported via the connection between the loading zone and the reaction zone to the reaction zone. Moreover, the reaction zone shall be also connected to a detector. The connection shall be such that the detector can detect the binding of the biomarkers to their detection agents. Suitable connections depend on the techniques used for measuring the presence or amount of the biomarkers. For example, for optical detection, transmission of light may be required between the detector and the reaction zone while for electrochemical determination a fluidal connection may be required, e.g., between the reaction zone and an electrode.

The detector shall be adapted to detect determination of the amount of the biomarkers. The determined amount can be subsequently transmitted to the evaluation unit. Said evaluation unit comprises a data processing element, such as a computer, with an implemented algorithm for determining the amount present in the sample.

The processing unit as referred to in accordance with the method of the present invention, typically, comprises a Central Processing Unit (CPU) and/or one or more Graphics Processing Units (GPUs) and/or one or more Application Specific Integrated Circuits (ASICs) and/or one or more Tensor Processing Units (TPUs) and/or one or more field-programmable gate arrays (FPGAs) or the like. A data processing element may be a general purpose computer or a portable computing device, for example. It should also be understood that multiple computing devices may be used together, such as over a network or other methods of transferring data, for performing one or more steps of the methods disclosed herein. Exemplary computing devices include desktop computers, laptop computers, personal data assistants ("PDA"), cellular devices, smart or mobile devices, tablet computers, servers, and the like. In general, a data processing element comprises a processor capable of executing a plurality of instructions (such as a program of software).

The evaluation unit, typically comprises or has access to a memory. A memory is a computer readable medium and may comprise a single storage device or multiple storage devices, located either locally with the computing device or accessible to the computing device across a network, for example. Computer-readable media may be any available media that can be accessed by the computing device and includes both volatile and nonvolatile media. Further, computer readable-media may be one or both of removable and non-removable media. By way of example, and not limitation, computer-readable media may comprise computer storage media. Exemplary computer storage media includes, but is not limited to, RAM, ROM, EEPROM, flash memory or any other memory technology, CD-ROM, Digital Versatile Disk (DVD) or other optical disk storage, magnetic cassettes, magnetic tape, magnetic disk storage or other magnetic storage devices, or any other medium which can be used for storing a plurality of instructions capable of being accessed by the computing device and executed by the processor of the computing device.

According to embodiments of the instant disclosure, software may include instructions which, when executed by a processor of the computing device, may perform one or more steps of the methods disclosed herein. Some of the instructions may be adapted to produce signals that control operation of other machines and thus may operate through those control signals to transform materials far removed from the computer itself. These descriptions and representations are the means used by those skilled in the art of data processing, for example, to most effectively convey the substance of their work to others skilled in the art.

The plurality of instructions may also comprise an algorithm which is generally conceived to be a self-consistent sequence of steps leading to a desired result. These steps are those requiring physical manipulations of physical quantities. Usually, though not necessarily, these quantities take the form of electrical or magnetic pulses or signals capable of being stored, transferred, transformed, combined, compared, and otherwise manipulated. It proves convenient at times, principally for reasons of common usage, to refer to these signals as values, characters, display data, numbers, or the like as a reference to the physical items or manifestations in which such signals are embodied or expressed. It should be borne in mind, however, that all of these and similar terms are to be associated with the appropriate physical quantities and are merely used here as convenient labels applied to these quantities.

The evaluation unit may also comprise or has access to an output device. Exemplary output devices include fax machines, displays, printers, and files, for example. According to some embodiments of the present disclosure, a computing device may perform one or more steps of a method disclosed herein, and thereafter provide an output, via an output device, relating to a result, indication, ratio or other factor of the method.

Typically, said measuring unit determines and comprises a detection system for a third biomarker, wherein said database comprises stored a stored reference for a third biomarker, said third biomarker being

(i) if a BMP10-type peptide is the second biomarker, CRP or at least one lipid biomarker selected from the group consisting of Cholesterol, TAG (Triglycerides), LDL (Low-density Lipoprotein), HDL (High-density Lipoprotein) and Apolipoprotein A-1,
(ii) if FGF23 is the second biomarker, a BMP10-type peptide, a BNP-type peptide, CRP, ANG2, or at least one lipid biomarker selected from the group consisting of Cholesterol, TAG (Triglycerides), LDL and HDL, or
(iii) if a BNP-type peptide is the second biomarker, Cholesterol or ANG2, or
(iv) if ANG-2 is the second biomarker, APOAT or LDL.

More typically, said detection system comprises at least one detection agent being capable of specifically detecting each of the biomarkers.

The present invention further contemplates a device for assessing myocardial infarction in a subject, said device comprising an evaluation unit comprising a database with stored references for a first biomarker being cMyBPC and a second biomarker, said second biomarker being a BMP10-type peptide, FGF23, a BNP-type peptide, GDF15, ANG2, CRP (C-reactive protein), ESM1, or a lipid biomarker, such as Cholesterol or LDL, and a data processor comprising instructions for carrying out a comparison of the amount of the first biomarker and the second biomarker to references, preferably, as specified above, and for assessing myocardial infarction based on the comparison, said evaluation unit being capable of receiving values for the amounts of the biomarkers determined in a sample of the subject, wherein, optionally, said database comprises a stored reference for a third biomarker, said third biomarker being

(i) if a BMP10-type peptide is the second biomarker, CRP or at least one lipid biomarker selected from the group consisting of Cholesterol, TAG (Triglycerides), LDL (Low-density Lipoprotein), HDL (High-density Lipoprotein) and Apolipoprotein A-1; or

(ii) if FGF23 is the second biomarker, a BMP10-type peptide, a BNP-type peptide, CRP, ANG2, or at least one lipid biomarker selected from the group consisting of Cholesterol, TAG (Triglycerides), LDL and HDL,

(iii) if a BNP-type peptide is the second biomarker, Cholesterol or ANG2,

(iv) if ANG-2 is the second biomarker, APOAT or LDL.

The present invention, in principle, also relates to the in vitro use of i) a first biomarker being cMyBPC and a second biomarker, said second biomarker being a BMP10-type peptide, FGF23, a BNP-type peptide, GDF15, ANG2, CRP (C-reactive protein), ESM1, or a lipid biomarker, such as Cholesterol or LDL, ii) or at least one detection agent for said first biomarker and at least one detection agent for said second biomarker for assessing myocardial infarction in a subject, wherein the assessment of myocardial infarction is i) the differentiation between type 1 and type 2 myocardial infarction, ii) the diagnosis of type 2 myocardial infarction, or iii) the guidance of myocardial infarction therapy.

The term "detection agent" as used herein refers to an agent that specifically detects the biomarker. In particular, the term refers to any agent which specifically binds to a biomarker, i.e. an agent which does not cross-react with other components present in the sample. Typically, a detection agent specifically binding a biomarker as referred to herein may be an antibody, an antibody fragment or derivative, an aptamer, a ligand for the biomarker, a receptor for the biomarker, an enzyme known to bind and/or convert the biomarker, or a small molecule known to specifically bind to the biomarker. For example, antibodies as referred to herein as detection agents include both polyclonal and monoclonal antibodies, as well as fragments thereof, such as Fv, Fab and F(ab)2 fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Aptamer detection agents, e.g., may be nucleic acid or peptide aptamers. Methods to prepare such aptamers are well-known in the art. For example, random mutations can be introduced into the nucleic acids or peptides being the basis for aptamers. These derivatives can then be tested for binding according to screening procedures known in the art, e.g. phage display. Specific binding of a detection agent means that it should not bind substantially to, i.e. cross-react with, another peptide, polypeptide or substance present in the sample to be analyzed. Preferably, the specifically bound biomarker should be bound with at least 3 times higher, more preferably at least 10 times higher and even more preferably at least 50 times higher affinity than any other components of the sample. Non-specific binding may be tolerable, if it can still be distinguished and measured unequivocally, e.g. according to its size on a Western Blot, or by its relatively higher abundance in the sample.

In a preferred embodiment, the detection agent is an antibody, or antigen-binding fragment thereof, which specifically binds the marker.

The detection agent may be fused or linked permanently or reversibly to a detectable label. Suitable labels are well known to the skilled artisan. Suitable detectable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include 35S, 125I, 32P, 33P and the like. A radioactive label can be detected by any method known and appropriate, e.g. a light-sensitive film or a phosphor imager. Suitable labels may also be or comprise tags, such as biotin, digoxygenin, His-Tag, Glutathion-S-Transferase, FLAG, GFP, myc-tag, influenza A virus haemagglutinin (HA), maltose binding protein, and the like.

The determination of lipid biomarkers by enzymatic assays is described elsewhere herein. Preferred detection agent are also disclosed for these markers.

The determination of a biomarker as set forth herein may comprise mass spectrometry (MS) which is carried out after the separation step (e.g. by LC or HPLC). Mass spectrometry as used herein encompasses all techniques which allow for the determination of the molecular weight (i.e. the mass) or a mass variable corresponding to a compound, i.e. a biomarker, to be determined in accordance with the present invention. Preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS, TOF or any combined approac-es using the aforementioned techniques. How to apply these techniques is well known to the person skilled in the art. Moreover, suitable devices are commercially available. More preferably, mass spectrometry as used herein relates to LC-MS and/or HPLC-MS, i.e. to mass spectrometry being operatively linked to a prior liquid chromatography separation step. Preferably, the mass spectrometry is tandem mass spectrometry (also known as MS/MS). Tandem mass spectrometry, also known as MS/MS involves two or more mass spectrometry step, with a fragmentation occurring in between the stages. In tandem mass spectrometry two mass spectrometers in a series connected by a collision cell. The mass spectrometers are coupled to the chromatographic device. The sample that has been separated by a chromatography is sorted and weighed in the first mass spectrometer, then fragmented by an inert gas in the collision cell, and a piece or pieces sorted and weighed in the second mass spectrometer. The fragments are sorted and weighed in the second mass spectrometer. Identification by MS/MS is more accurate.

In an embodiment, mass spectrometry as used herein encompasses quadrupole MS. Most preferably, said quadrupole MS is carried out as follows: a) selection of a mass/charge quotient (m/z) of an ion created by ionisation in a first analytical quadrupole of the mass spectrometer, b) fragmentation of the ion selected in step a) by applying an acceleration voltage in an additional subsequent quadrupole which is filled with a collision gas and acts as a collision chamber, c) selection of a mass/charge quotient of an ion created by the fragmentation process in step b) in an additional subsequent quadrupole, whereby steps a) to c) of the method are carried out at least once and analysis of the mass/charge quotient of all the ions present in the mixture of substances as a result of the ionisation process, whereby the quadrupole is filled with collision gas but no acceleration voltage is applied during the analysis. Details on said most preferred mass spectrometry to be used in accordance with the present invention can be found in WO2003/073464.

More preferably, said mass spectrometry is liquid chromatography (LC) MS such high performance liquid chromatography (HPLC) MS, in particular HPLC-MS/MS. Liquid chromatography as used herein refers to all techniques which allow for separation of compounds (i.e. metabolites) in liquid or supercritical phase.

For mass spectrometry, the analytes in the sample are ionized in order to generate charged molecules or molecule fragments. Afterwards, the mass-to-charge of the ionized analyte, in particular of the ionized biomarkers, or fragments thereof is measured. Prior to the ionization, the sample may be subjected to cleavage with a protease, e.g. with trypsin. The protease cleaves the protein biomarkers into smaller fragments.

Thus, the mass spectrometry step preferably comprises an ionization step in which the biomarkers to be determined are ionized. Of course, other compounds present in the sample/elulate are ionized as well. Ionization of the biomarkers can be carried out by any method deemed appropriate, in particular by electron impact ionization, fast atom bombardment, electrospray ionization (ESI), atmospheric pressure chemical ionization (APCI), matrix assisted laser desorption ionization (MALDI).

In a preferred embodiment, the ionization step (for mass spectrometry) is carried out by electrospray ionization (ESI). Accordingly, the mass spectrometry is preferably ESI-MS (or if tandem MS is carried out: ESI-MS/MS). Electrospray is a soft ionization method which results in the formation of ions without breaking any chemical bonds.

More typically, a third biomarker or at least one detection agent for said third biomarker is used in addition, said third biomarker being

(i) if a BMP10-type peptide is the second biomarker, CRP or at least one lipid biomarker selected from the group consisting of Cholesterol, TAG (Triglycerides), LDL (Low-density Lipoprotein), HDL (High-density Lipoprotein) and Apolipoprotein A-1,
(ii) if FGF23 is the second biomarker, a BMP10-type peptide, a BNP-type peptide, CRP, ANG2, or at least one lipid biomarker selected from the group consisting of Cholesterol, TAG (Triglycerides), LDL and HDL, or
(iii) if a BNP-type peptide is the second biomarker, Cholesterol or ANG2, or
(iv) if ANG-2 is the second biomarker, APOAT or LDL.

The present invention also relates to a kit for assessing myocardial infarction in a subject, said kit comprising at least one antibody, or antigen-binding fragment thereof which specifically binds to a first biomarker being cMyBPC and at least one antibody, or antigen-binding fragment thereof which specifically binds to a second biomarker, said second biomarker being a BMP10-type peptide, FGF23, a BNP-type peptide, GDF15, ANG2, CRP (C-reactive protein), ESM1, or a lipid biomarker, such as Cholesterol or LDL, wherein optionally, said kit further comprises a detection agent for a third biomarker, said third biomarker being (i) if a BMP10-type peptide is the second biomarker, CRP, ANG2 or at least one lipid biomarker selected from the group consisting of Cholesterol, TAG (Triglycerides), LDL (Low-density Lipoprotein), HDL (High-density Lipoprotein) and Apolipoprotein A-1, or (ii) if FGF23 is the second biomarker, a BMP10-type peptide, a BNP-type peptide, CRP, ANG2, or at least one lipid biomarker selected from the group consisting of Cholesterol, TAG (Triglycerides), LDL and HDL, or (iii) if a BNP-type peptide is the second biomarker, Cholesterol or ANG2, (iv) if ANG2 is the second biomarker, APOAT or LDL, wherein the assessment of myocardial infarction is i) the differentiation between type 1 and type 2 myocardial infarction, ii) the diagnosis of type 2 myocardial infarction, or iii) the guidance of myocardial infarction therapy.

The term "kit" as used herein refers to a collection of the aforementioned components, typically, provided in separate or within a single container. The container also typically comprises instructions for carrying out the method of the present invention. These instructions may be in the form of a manual or may be provided by a computer program code which is capable of carrying out or supports the determination of the biomarkers referred to in the methods of the present invention when implemented on a computer or a data processing device. The computer program code may be provided on a data storage medium or device such as an optical storage medium (e.g., a Compact Disc) or directly on a computer or data processing device or may be provided in a download format such as a link to an accessible server or cloud. Moreover, the kit may, usually, comprise standards for reference amounts of biomarkers for calibration purposes as described elsewhere herein in detail. The kit according to the present invention may also comprise further components which are necessary for carrying out the method of the invention such as solvents, buffers, washing solutions and/or reagents required for detection of the released second molecule. Further, it may comprise the device of the invention either in parts or in its entirety.

More typically, said kit further comprises at least one detection agent for a third biomarker, said third biomarker being

(i) if a BMP10-type peptide is the second biomarker, CRP or at least one lipid biomarker selected from the group consisting of Cholesterol, TAG (Triglycerides), LDL (Low-density Lipoprotein), HDL (High-density Lipoprotein) and Apolipoprotein A-1, or

(ii) if FGF23 is the second biomarker, a BMP10-type peptide, a BNP-type peptide, CRP, ANG2, or at least one lipid biomarker selected from the group consisting of Cholesterol, TAG (Triglycerides), LDL and HDL,

(iii) if a BNP-type peptide is the second biomarker, Cholesterol or ANG2,

(iv) if ANG-2 is the second biomarker, APOAT or LDL.

It is to be understood that the definitions and explanations of the terms made above apply accordingly for all embodiments described in this specification and the accompanying claims.

EXAMPLES

The Examples shall merely illustrate the invention.

## 1. Determination of biomarkers

## 1.1. Determination of biomarkers by sandwich assays on cobas Elecsys® ECLIA platform (ECLIA Assays from Roche Diagnostics, Germany)

The Elecsys® Electro- ChemiLuminescence (ECL) technology and assay method is briefly described below for the determination of GDF-15. The concentration of GDF-15 was determined by a cobas e801 analyzer. Detection of GDF-15 with a cobas e801 analyzer is based on the Elecsys® Electro-ChemiLuminescence (ECL) technology. In brief, biotin-labelled and ruthenium-labelled antibodies are combined with the respective amount of undiluted sample and incubated on the analyzer. Subsequently, streptavidin-coated magnetic microparticles are added and incubated on the instrument in order to facilitate binding of the biotin-labelled immunological complexes. After this incubation step the reaction mixture is transferred into the measuring cell where the beads are magnetically captured on the surface of an electrode. ProCell M Buffer containing tripropylamine (TPA) for the subsequent ECL the reaction is then introduced into the measuring cell in order to separate bound immunoassay complexes from the free remaining particles. Induction of voltage between the working and the counter electrode then initiates the reaction leading to emission of photons by the ruthenium complexes as well as TPA. The resulting electrochemiluminescent signal is recorded by a photomultiplier and converted into numeric values indicating concentration level of the respective analyte.

cTNThs (high-sensitive cTroponinT), NTpBNP (N-terminal prohormone of brain natriuretic peptide), GDF15 (Growth/differentiation factor 15) , cMyBPC (cardiac Myosin binding protein C), BMP10 (Bone Morphogenic Protein 10-type peptide), FGF23 (Fibroblast growth factor 23), ANG2 (Angiopoietin2) and ESM-1 (endothelial cell specific molecule 1) were measured in EDTA plasma samples with sandwich immunoassays.

cTNThs (high-sensitive cTroponinT), NTpBNP (N-terminal prohormone of brain natriuretic peptide) and GDF15 (Growth/differentiation factor 15) were measured with commercial ECLIA assays which were developed for the cobas Elecsys® ECLIA platform (ECLIA Assays from Roche Diagnostics, Germany) in EDTA plasma samples according to the manufactures' instructions on a cobas Elecsys® ECLIA immunoassay platform (Roche Diagnostics, Germany).

The following assays were used:

cTNThs: (Troponin T hs Elecsys G5), electrochemiluminescence immunoassay.

NTproBNP: (proBNP-II Elecsys), electrochemiluminescence immunoassay.

GDF15: (GDF-15 Elecsys), electrochemiluminescence immunoassay.

cMyBPC (cardiac Myosin binding protein C), BMP10 (Bone Morphogenic Protein 10-type peptide), FGF23 (Fibroblast growth factor 23), ANG2 (Angiopoietin2) and ESM-1 (endothelial cell specific molecule 1) were measured with robust prototype ECLIA assays. These sandwich-immunoassays were developed in-house for the cobas Elecsys® ECLIA platform (ECLIA Assay from Roche Diagnostics, Germany). The assays comprise a biotinylated and a ruthenylated monoclonal antibody that specifically binds to the analyte from the EDTA plasma samples and measured on a cobas Elecsys® ECLIA immunoassay platform analyzer (Roche Diagnostics, Germany). For example, an assay was used for BMP10 that uses antibodies binding to NT-proBMP10. Thus, the assay detects NT-proBMP10 and BMP10-type peptides comprising the NT-proBMP10 sequence.

**1.2. Determination of biomarkers on cobas® clinical chemistry analyzer platform (Roche Diagnostics, Germany)**

CRPhs (high sensitive C reactive Protein), CysC2 (Cystatin C) and the lipid biomarker CHOL (Cholesterol), TRIGL (triglycerides), LDL (Low-density Lipoprotein), HDL (High-density Lipoprotein), and APOAT (Apolipoprotein A-1) were measured by using commercial assays (Roche Diagnostics, Germany) in EDTA plasma samples according to the manufactures' instructions on the cobas® clinical chemistry analyzer platform cobas c 501 (Roche Diagnostics, Germany).

The following assays were used:

CRPhs: (Cat. No. 04628918 190 Cardiac-C reactive Protein (Latex) hs), Particle enhanced immunoturbidimetric assay

APOAT: (Cat. No. 03032566 Tina quant ApolipoproteinA-1 ver 2), immunoturbidimetric assay

CHOL: (Cat. No. 03039773 Cholesterol Gen.2), enzymatic colorimetric assay

HDL: (Cat. No. 07528566 HDL-C Gen), homogeneous enzymatic colorimetric assay

LDL: (Cat. No. 07005717 LDL-C Gen3), homogeneous enzymatic colorimetric assay

TRIGL: (Cat. No. 20767107 Triglycerides), enzymatic colorimetric assay

**2. Patient cohort, APACE Study**

The Advantageous Predictors of Acute Coronary Syndrome Evaluation (APACE) study is described in Nestelberger et al JAMA Cardiol. 2021;6(7):771-780. doi:10.1001/jamacardio.2021.0669. In short, the study is a international multicenter prospective cohort study to enroll unselected patients presenting with acute chest pain at rest within the last 12 hours to the emergency department (ED) and registered on ClinicalTrials.gov (identifier: NCT00470587). Diagnosis of acute myocardial infarction (AMI) is performed in consecutively enrolled patients against a clinical reference standard (final diagnosis) with central adjudication by 2 independent cardiologists according to the universal definition of AMI (Thygesen K et al, Eur Heart J. 2019;40:226), using all clinical information, including cardiac imaging and serial measurements of high sensitive troponins.

AMI was diagnosed when there was evidence of myocardial necrosis in association with a clinical setting consistent with myocardial ischemia. Myocardial necrosis was diagnosed by at least one hs-cTnT/I value above the 99th percentile together with a significant rise and/or fall. Absolute changes in high-sensitivity troponin T and I (hs-cTnT/I) were used to determine significant changes based on the diagnostic superiority of absolute over relative changes. All other patients were classified in the categories of unstable angina, non-cardiac chest pain, cardiac but non-coronary disease (e.g. myocarditis, takotsubo syndrome, heart failure), and symptoms of unknown origin with normal hs-cTnT/I levels.

Definition of type 1 and type 2 myocardial infarction:

Type 1 AMI (TIMI) and Type 2 AMI (T2MI) were defined according to the universal definition of AMI (4th edition, Thygesen K et al, Eur Heart J. 2019;40:226). In short, both diagnoses (T1 and T2 AMI) require clinical evidence of acute myocardial ischemia, such as changes in cardiac troponin, but with atherothrombotic origin for T1MI, and non-atherothrombotic origin for T2MI. In more detail, the evidence of myocardial necrosis in a clinical setting consistent with acute myocardial ischemia, Type 1 MI was defined as spontaneous MI related to a primary atherothrombotic coronary event such as plaque erosion or rupture, intraluminal coronary thrombus, or distal microembolization. Type 2 MI was defined as secondary due to an oxygen supply-demand mismatch. Conditions reflecting an imbalance between myocardial oxygen supply and demand, included brady- or tachyarrhythmias, hypoxaemia, hypotension, hypertension, severe anemia, coronary artery spasm, dissection, and coronary embolism. Underlying coronary artery disease was possible, but not required for the diagnosis of T2MI. To qualify for T2MI, the same dynamic changes in hs-cTnT/I were required as for TIMI. As recommended, the documentation of a clear trigger was essential for the diagnosis of T2MI. Coronary angiography was not mandatory for a diagnosis of TIMI in order to limit the possible effect of a selection bias due to clinical referral to coronary angiography. No other subtypes of MI were reported.

**Example 1: Patients with AMI from the APACE study**

From a total of 4216 patients from the APACE study, N = 874 patients were diagnosed with AMI (20.7 %). Out of these, 708 patients were diagnosed with Type 1 MI (564 patients with Non ST elevation AMI, 144 patients with ST elevation myocardial infarction) and 166 patients were diagnosed with Type 2 MI (164 patients with Non ST elevation myocardial infarction, 2 patients with ST elevation myocardial infarction).

- Case (N=166): Patients with Type 2 MI secondary to ischemia due to either increased oxygen demand or decreased supply. Out of these 58 patients were female and 108 patients were male.

- Control (N=708): Patients with Type 1 MI spontaneous MI related to ischemia due to a primary coronary event such as plaque erosion and or rupture, fissuring or dissection. Out of these 191 patients were female and 517 patients were male.

Marker values were transformed with the logarithm to the base 2 and mathematically combined via logistic regression. The "area under the receiver operating characteristic curve" (AUC) was used as a general measure for marker performance.

**Biomarkers are differently elevated in type 1 or in type 2 myocardial infarction**

**Table 1: Univariate AUCs of biomarkers presented and their average dynamics (elevated or decreased) in Type 1 and Type 2 MI patients**

| Biomarker | AUC | Direction |
|---|---|---|
| hsTnT | 67.784 | Elevated in Type 1 MI, decreased in Type 2 MI |
| cMyBPC | 70.754 | Elevated in Type 1 MI, decreased in Type 2 MI |
| LDL | 59.294 | Elevated in Type 1 MI, decreased in Type 2 MI |
| TRIGL | 56.054 | Elevated in Type 1 MI, decreased in Type 2 MI |
| CHOL | 59.611 | Elevated in Type 1 MI, decreased in Type 2 MI |
| ANG2 | 52.237 | Elevated in Type 2 MI, decreased in Type 1 MI |
| BMP10 | 60.118 | Elevated in Type 2 MI, decreased in Type 1 MI |

(continued)

| Biomarker | AUC | Direction |
|---|---|---|
| ESM1 | 55.674 | Elevated in Type 2 MI, decreased in Type 1 MI |
| FGF23 | 58.982 | Elevated in Type 2 MI, decreased in Type 1 MI |
| tproBNP | 53.149 | Elevated in Type 2 MI, decreased in Type 1 MI |
| proBNP | 52.750 | Elevated in Type 2 MI, decreased in Type 1 MI |
| GDF15 | 58.282 | Elevated in Type 2 MI, decreased in Type 1 MI |
| APOAT | 50.423 | Elevated in Type 2 MI, decreased in Type 1 MI |
| CRPhs | 48.772 | Elevated in Type 2 MI, decreased in Type 1 MI |
| HDL | 53.856 | Elevated in Type 2 MI, decreased in Type 1 MI |

Combinations of marker pairs (bivariate marker combinations) and marker triplets (trivariate marker combinations) having improved AUCs over the single marker cMyBPC are shown in **Table 2.** Combinations of marker pairs (bivariate marker combinations) and marker triplets (trivariate marker combinations) *not* having improved AUCs over the single marker cMyBPC are shown in **Table 3.**

**Table 2:** The univariate performance of cMyBPC (AUC) **considering all patient subgroups** and combinations of cMyBPC with a second marker (bivariate marker combinations) and a third marker (trivariate marker combinations) having improved AUCs over the single marker cMyBPC by at least an improvement of **2.0** of the AUC (Impr. AUC).

| cMyBPC and marker combinations | | | | |
|---|---|---|---|---|
| Marker 1 | Marker 2 | Marker 3 | AUC | Impr. AUC |
| cMyBPC | - | - | 70.754 | |
| cMyBPC | BMP10 | - | 75.676 | 4.922 |
| cMyBPC | FGF23 | - | 75.674 | 4.920 |
| cMyBPC | NTproBNP | - | 75.129 | 4.375 |
| cMyBPC | FNTproBNP | - | 74.608 | 3.853 |
| cMyBPC | DF15 | - | 74.328 | 3.574 |
| cMyBPC | ANG2 | - | 73.873 | 3.119 |
| cMyBPC | Chol | - | 73.827 | 3.073 |
| cMyBPC | ESM1 | - | 73.111 | 2.357 |
| cMyBPC | CRPhs | - | 72.733 | 1.979 |
| cMyBPC | LDL | - | 72.855 | 2.101 |
| cMyBPC | BMP10 | CHOL | 77.592 | 6.838 |
| cMyBPC | BMP10 | LDL | 76.993 | 6.239 |
| cMyBPC | BMP10 | TRIGL | 76.516 | 5.762 |
| cMyBPC | BMP10 | CRPhs | 76.411 | 5.657 |
| cMyBPC | BMP10 | APOAT | 76.012 | 5.258 |
| cMyBPC | BMP10 | ANG2 | 75.966 | 5.212 |
| cMyBPC | BMP10 | HDL | 75.634 | 4.880 |
| cMyBPC | FGF23 | TRIGL | 76.771 | 6.017 |
| cMyBPC | FGF23 | BMP10 | 77.169 | 6.415 |
| cMyBPC | FGF23 | NTproBNP | 76.935 | 6.181 |
| cMyBPC | FGF23 | NTproBNP | 76.442 | 5.688 |

(continued)

| cMyBPC and marker combinations | | | | |
|---|---|---|---|---|
| Marker 1 | Marker 2 | Marker 3 | AUC | Impr. AUC |
| cMyBPC | FGF23 | CHOL | 76.766 | 6.012 |
| cMyBPC | FGF23 | HDL | 76.290 | 5.536 |
| cMyBPC | FGF23 | LDL | 76.243 | 5.489 |
| cMyBPC | FGF23 | CRPhs | 76.051 | 5.297 |
| cMyBPC | FGF23 | ANG2 | 76.011 | 5.257 |
| cMyBPC | NTproBNP | CHOL | 76.314 | 5.560 |
| cMyBPC | NTproBNP | ANG2 | 75.516 | 4.762 |
| cMyBPC | ANG2 | APOAT | 73.974 | 3.220 |
| cMyBPC | ANG2 | LDL | 74.945 | 4.191 |
| | | | | |

**Table 2** summarizes the performance of the single biomarker cMyBPC versus marker combinations including cMyBPC in patients of the APACE study.

Results shown in Table 2 show the incremental value of using combinations of biomarkers in the assessment of T1MI versus T2MI.

Interestingly combinations of cMyBPC with a second biomarker selected out of several biomarkers indicating vascular alterations comprising BMP10, FGF23, NTproBNP, tNTproBNP, GDF15, ANG2, Cholesterol (CHOL) or ESM1 are found with improved performance versus the single marker cMyBPC by delta AUC changes above 2 (4.922, 4.920, 4.375, 3.853, 3.574, 3.119, 3.073 or 2.357 respectively).

Marker combinations of cMyBPC including a third biomarker even improve the performance by delta AUC changes up to 6.8.

The choice of BMP-10, FGF-23 or a BNP type protein (NTproBNP or tNTproBNP) as second biomarker is particularly useful in the diagnostic evaluation of Type 1 MI versus Type 2 MI with observed AUC delta changes versus cMyBPC of 4.922, 4.920 or (4.375 or 3.853) respectively.

It is concluded from the data above that the addition of a third biomarker further improves the assessment of Type 1 MI versus Type 2 MI (delta AUC changes up to 6.8 versus the single biomarker cMyBPC).

A surprising finding relates to the beneficial effects of lipid biomarkers selected as third biomarkers to the marker combination of cMyBPC and BMP10 (e.g. CHOL, LDL, TRIGL, HDL or APOAT).

As shown in the table above, the choice of CHOL, LDL, TRIGL, CRPhs, APOAT, ANG2 or HDL in addition to the combination of BMP10 and cMyBPC leads to a further meaningful improvement versus the single marker cMyBPC by delta changes of AUC 6.838, 6.239, 5.762, 5.657, 5.258, 5.212 or 4.880 (AUC 70.754 $\Rightarrow$ AUC 77.592, 76.993, 76.516, 76.411, 76.012, 75.966 or 75.634 respectively).

A surprising finding relates to the beneficial effects of lipid biomarkers selected as third biomarkers to the marker combination of cMyBPC and FGF23 (e.g. CHOL, LDL, or HDL).

As shown in the table above, the choice of BMP10, NTproBNP, tNTproBNP, CHOL, HDL, LDL, CRPhs or ANG2 in addition to the combination of FGF23 and cMyBPC leads to a further meaningful improvement versus the single marker cMyBPC (delta changes of AUC 6.415, 6.181, 5.688, 6.012, 5.536, 5.489, 5.297 or 5.257; AUC 70.754 $\Rightarrow$ AUC 77.169, 76.935, 76.442, 76.766, 76.290, 76.243, 76.051 or 76.011 respectively).

Next the choice of cMyBPC as first biomarker, NTproBNP as second biomarker and Chol or ANG2 as third biomarker results in an improved performance versus the single marker cMyBPC (AUC 70.754 $\Rightarrow$ 76.314 or 75.516 respectively).

In summary it is beneficial to improve the assessment of Type 1 MI versus Type 2 MI by adding at least a second biomarker selected out of BMP10, FGF23 or a BNP-type marker to cMyBPC. Even more improved performances can be achieved with a third biomarker selected out of lipid markers, e.g. CHOL, LDL, TRIGL, APOAT or HDL) or ANG2 or hsCRP.

It is of particular interest that the data above provide strong evidence for the beneficial effects of lipid parameters in panels comprising either cMyBPC and BMP10 or cMyBPC and FGF23 for the assessment of Type 1 MI versus Type 2 MI.

**Table 3:** The univariate performance of cMyBPC (AUC) considering all patient subgroups and combinations of cMyBPC with a second marker (bivariate marker combinations) and a third marker (trivariate marker combinations) *not* having an improved AUC of at least 2.0 (Impr. AUC) over the single biomarker cMyBPC.

| cMyBPC and marker combinations | | | | |
|---|---|---|---|---|
| Marker 1 | Marker 2 | Marker 3 | AUC | Impr. AUC |
| cMyBPC | - | - | 70.754 | |
| cMyBPC | hsTnT | - | 70.956 | 0.202 |
| MyBPC | GLUC | - | 70.789 | 0.035 |
| MyBPC | CysC | - | 71.025 | 0.271 |
| cMyBPC | APOAT | - | 71.036 | 0.282 |
| cMyBPC | HDL | - | 70.874 | 0.119 |
| cMyBPC | hsTnT | CvsC | 71.370 | 0.615 |
| cMyBPC | GLUC | HDL | 70.837 | 0.083 |
| cMyBPC | TRIGLY | | 72.398 | 1.644 |
| cMyBPC | IGFBP7 | | 72.211 | 1.456 |

As shown in Table 3 several biomarker combinations do not improve the AUC value over the single marker cMyBPC for the assessment of Type1 MI versus Type 2 MI.

**Example 2: Example of use for a cMyBPC and BMP10 combination**

The following example illustrates how in practice a score can be obtained from the measured biomarker concentrations from a patient with a Type 1 MI and a patient with a Type 2 MI respectively.

Let $cMyBPC_{Type1}$ and $BMP10_{Type1}$ denote the concentrations obtained from the assays measuring cMyBPC and BMP10 respectively of a Type 1 AMI patient. Let $cMyBPC_{Type2}$ $cMyBPC_{Type2}$ denote the concentrations obtained from the assays measuring cMyBPC and BMP10 respectively of a Type 2 AMI patient. Let $\beta_0$ denote the offset and $\beta_{cMyBPC}$ and $\beta_{BMP10}$ denote the weighting factors (coefficients) of the mathematical model used to combine the obtained concentrations.

Further, let $\alpha$ denote a predetermined cutoff, which optimally separates Type 1 and Type 2 AMI patients.

The score of the measured biomarker concentrations for the type 1 AMI patient, denoted as $score_{Type1}$, is obtained by computing

$$\text{score}_{Type1} = \beta_0 + \beta_{cMyBPC} \log_2(cMyBPC_{Type1}) + \beta_{BMP10} \log_2(BMP10_{Type1}).$$

Here $\log_2( )$ denotes the logarithm function to the base 2.

The score of the measured biomarker concentrations for the type 2 AMI patient, denoted as $score_{Type1}$, is obtained by computing

$$\text{score}_{Type2} = \beta_0 + \beta_{cMyBPC} \log_2(cMyBPC_{Type2}) + \beta_{BMP10} \log_2(BMP10_{Type2}).$$

Should the obtained score be larger than the predetermined value of the cutoff $\alpha$, it is suggested that the patient has suffered a Type 2 AMI. Should the obtained score be smaller than the predetermined value of the cutoff $\alpha$, it is suggested that the patient has suffered a Type 1 AMI.

**Example 3: Patients with Diabetes from the APACE study**

- Case (N=166): Patients with Type 2 MI secondary to ischemia due to either increased oxygen demand or decreased supply. Out of these 37 patients had a history of Diabetes and 129 patients not.

- Control (N=708): Patients with Type 1 MI spontaneous MI related to ischemia due to a primary coronary event such as plaque erosion and or rupture, fissuring or dissection. Out of these 196 patients had a history of Diabetes and 512 patients not.

**Table 4:** Patients with Type 1 MI with and without a history of diabetes and patients with type 2 MI with and without a history of diabetes

| | Type 1 MI | | Type 2 MI | |
|---|---|---|---|---|
| | n all | % all | n all | % all |
| History Diabetes: no | 512 | 0.72 | 129 | 0.78 |
| History Diabetes: yes | 196 | 0.28 | 37 | 0.22 |

**Table 5:** The univariate performance of cMyBPC (AUC) considering all patients with a history of diabetes and combinations of cMyBPC with a second marker (bivariate marker combinations) and a third marker (trivariate marker combinations). All bivariate and trivariate combinations have at least a 3.0 improvement in AUC in patients with history of diabetes versus patients with no history of diabetes. All bivariate and trivariate combinations having at least a 3.0 improvement in AUC versus the univariate performance of cMyBPC in both subgroups are reported for markers used in combinations for reference.

| Marker combinations | | | Patient subgroups | | | |
|---|---|---|---|---|---|---|
| Marker 1 | Marker 2 | Marker 3 | Diabetic | | Non-Diabetic | |
| | | | AUC | Impr. AUC | AUC | Impr. AUC |
| cMyBPC | - | - | 73.277 | | 70.208 | |
| cMyBPC | ANG2 | - | 76.881 | 3.604 | 73.531 | 3.323 |
| cMyBPC | ANG2 | AOPAT | 76.829 | 3.552 | 73.624 | 3.416 |
| cMyBPC | ANG2 | LDL | 78.378 | 5.101 | 74.876 | 4.668 |

**Table 5** provides the performance of marker combinations in patients with a history of Diabetes versus patients without. Table 5 shows an improved performance of cMyBPC in combination with several biomarker panels. It is particularly interesting that biomarker panels improving the performance of cMyBPC comprise ANG2. In patients with Diabetes biomarker panels of cMyBPC and ANG2 show an improvement versus cMyBPC by meaningful AUC delta changes of 3.604 (AUC 73.277 ⇒AUC 76.881). Adding a third biomarker LDL to cMyBPC and ANG2 results in a further improvement versus cMyBPC by a AUC delta change of 5.101 (AUC 73.277 ⇒AUC 78.378) in the subgroup of patients with Diabetes.

As concluded from these results it is beneficial to use biomarker combinations of cMyBPC with the second biomarker ANG2 in the subgroup of patients with Diabetes.

**Table 6:** The univariate performance of cMyBPC (AUC) considering all patients with a history of diabetes and combinations of cMyBPC with a second marker (bivariate marker combinations) and a third marker (trivariate marker combinations). All bivariate and trivariate combinations do *not* have at least a 3.0 improvement in AUC in patients with history of diabetes versus patients with no history of diabetes. Univariate performance in both subgroups are reported for markers used in combinations for reference.

| cMyBPC and marker combinations | | | AUC | |
|---|---|---|---|---|
| Marker 1 | Marker 2 | Marker 3 | Diabetic | Non-Diabetic |
| cMyBPC | - | - | 73.277 | 70.208 |
| ANG2 | - | - | 59.886 | 49.055 |
| sFlt1 | - | - | 52.868 | 53.880 |
| LDL | - | - | 63.408 | 59.599 |
| APOAT | - | - | 56.196 | 51.748 |
| cMyBPC | APOAT | - | 73.831 | 70.412 |

**Claims**

1. An in vitro method for assessing myocardial infarction in a subject:

   (a) determining the amount of a first biomarker in a sample of the subject, said first biomarker being cMyBPC (cardiac Myosin binding protein C);
   (b) determining the amount of a second biomarker in a sample of the subject, said second biomarker being a lipid biomarker, such as Cholesterol or LDL (Low Density Lipoprotein), a BMP10-type peptide (Bone Morphogenic Protein 10-type peptide), FGF23 (Fibroblast growth factor 23), a BNP-type peptide (Brain natriuretic peptide type peptide), GDF-15 (Growth differentiation factor 15), ANG2 (Angiopoietin 2), or CRP (C-reactive protein), ESM1 (endothelial cell specific molecule 1);
   (c) comparing the amounts of the biomarkers to references for said biomarkers and/or calculating a score for assessing myocardial infarction based on the amounts of the biomarkers; and
   (d) assessing myocardial infarction based on the comparison and/or the calculation made in step (c),

   wherein the assessment of myocardial infarction is i) the differentiation between type 1 and type 2 myocardial infarction, ii) the diagnosis of type 2 myocardial infarction, or iii) the guidance of myocardial infarction therapy.

2. The method of claim 1, wherein in step (b)

(i) if the amount of a BMP 10-type peptide is determined as the second biomarker, the method further comprises determining the amount of at least one lipid biomarker selected from the group consisting of Cholesterol, TAG (Triglycerides), LDL (Low-density Lipoprotein), HDL (High-density Lipoprotein) and Apolipoprotein A-1 or the amount of CRP or ANG2 as a third biomarker; or
(ii) if the amount of FGF23 is determined as the second biomarker, the method further comprises determining the amount of at least one lipid biomarker selected from the group consisting of Cholesterol, TAG (Triglycerides), LDL and HDL, or a BMP10-type peptide, a BNP-type peptide, CRP, or ANG2 as a third biomarker, or
(iii) if the amount of a BNP-type peptide is determined as the second biomarker, the method further comprises determining the amount of Cholesterol or ANG2, or
(iv) if the amount of ANG2 is determined as the second biomarker, the method further comprises determining the amount of or LDL or APOAT as a third biomarker

3. The method of claim 1 or 2, wherein the sample has been obtained from a subject at presentation at the emergency department.

4. The method of any one of claims 1 to 3, wherein said sample is a blood, serum or plasma sample.

5. The method of any one of claims 1 to 4, the subject is human.

6. A computer-implemented method for assessing myocardial infarction in a subject, said method comprising the steps of:

(a) receiving a value for the amount of a first biomarker in a sample of the subject, said first biomarker being cMyBPC;
(b) receiving a value for the amount of a second biomarker in a sample of the subject, said second biomarker being a BMP10-type peptide, FGF23, a BNP-type peptide, GDF15, ANG2, CRP (C-reactive protein), ESM1, or a lipid biomarker, such as Cholesterol or LDL;
(c) comparing the values for the amounts of the biomarkers to references for said biomarkers and/or calculating a score for assessing myocardial infarction based on the amounts of the biomarkers; and
(d) assessing myocardial infarction based on the comparison and/or the calculation made in step (c),

wherein the assessment of myocardial infarction is i) the differentiation between type 1 and type 2 myocardial infarction, ii) the diagnosis of type 2 myocardial infarction, or iii) the guidance of myocardial infarction therapy.

7. The method of claim 6, wherein in step (b)

(i) if the value for the amount of a BMP10-type peptide is received as the second biomarker, the method further comprises receiving a value for the amount of CRP, ANG2 or at least one lipid biomarker selected from the group consisting of Cholesterol, TAG (Triglycerides), LDL (Low-density Lipoprotein), HDL (High-density Lipoprotein) and Apolipoprotein A-1 as a third biomarker; or
(ii) if the value for the amount of FGF23 is received as the second biomarker, the method further comprises receiving a value for the amount of a BMP10-type peptide, a BNP-type peptide, CRP, ANG2, or at least one lipid biomarker selected from the group consisting of Cholesterol, TAG (Triglycerides), LDL and HDL as a third biomarker, or
(iii) if the value for the amount of a BNP-type peptide is received as the second biomarker, the method further comprises receiving a value for the amount of Cholesterol or ANG2, or
(iv) if the value for the amount of a ANG2 is received as the second biomarker, the method further comprises receiving a value for the amount of APOAT or LDL as a third biomarker.

8. A device for assessing myocardial infarction in a subject, said device comprising an evaluation unit comprising a database with stored references for a first biomarker being cMyBPC and a second biomarker, said second biomarker being a BMP10-type peptide, FGF23, a BNP-type peptide, GDF15, ANG2, CRP (C-reactive protein), ESM1, or a lipid biomarker, such as Cholesterol or LDL, and a data processor comprising instructions for carrying out a comparison of the amount of the first biomarker and the second biomarker to references, preferably, as specified in any one of claims 1 to 7 and for assessing myocardial infarction based on the comparison, said evaluation unit being capable of receiving values for the amounts of the biomarkers determined in a sample of the subject,

wherein, optionally, said database comprises a stored reference for a third biomarker, said third biomarker being

(i) if a BMP10-type peptide is the second biomarker, CRP, ANG2 or at least one lipid biomarker selected from the group consisting of Cholesterol, TAG (Triglycerides), LDL (Low-density Lipoprotein), HDL (High-density Lipoprotein) and Apolipoprotein A-1;
(ii) if FGF23 is the second biomarker, a BMP10-type peptide, a BNP-type peptide, CRP, ANG2, or at least one lipid biomarker selected from the group consisting of Cholesterol, TAG (Triglycerides), LDL and HDL, or
(iii) if a BNP-type peptide is the second biomarker, Cholesterol or ANG2, or
(iv) if ANG2 is the second biomarker, APOAT or LDL,

wherein the assessment of myocardial infarction is i) the differentiation between type 1 and type 2 myocardial infarction, ii) the diagnosis of type 2 myocardial infarction, or iii) the guidance of myocardial infarction therapy.

9. The in vitro use of i) a first biomarker being cMyBPC and a second biomarker, said second biomarker being a BMP10-type peptide, FGF23, a BNP-type peptide, GDF15, ANG2, CRP (C-reactive protein), ESM1, or a lipid biomarker, such as Cholesterol or LDL, or ii) at least one detection agent for said first biomarker, at least one detection agent for said second biomarker, for assessing myocardial infarction in a subject, wherein the assessment of myocardial infarction is i) the differentiation between type 1 and type 2 myocardial infarction, ii) the diagnosis of type 2 myocardial infarction, or iii) the guidance of myocardial infarction therapy.

10. The use of claim 9, wherein a third biomarker or a detection agent for a third biomarker is used in addition, said third biomarker being

(i) if a BMP10-type peptide is the second biomarker, CRP, ANG2 or at least one lipid biomarker selected from the group consisting of Cholesterol, TAG (Triglycerides), LDL (Low-density Lipoprotein), HDL (High-density Lipoprotein) and Apolipoprotein A-1,
(ii) if FGF23 is the second biomarker, a BMP10-type peptide, a BNP-type peptide, CRP, ANG2, or at least one lipid biomarker selected from the group consisting of Cholesterol, TAG (Triglycerides), LDL and HDL,
(iii) if a BNP-type peptide is the second biomarker, Cholesterol or ANG2, or
(iv) if ANG2 is the second biomarker, APOAT or LDL.

11. A kit for assessing myocardial infarction in a subject, said kit comprising at least one antibody, or antigen-binding fragment thereof which specifically binds to a first biomarker being cMyBPC and at least one antibody, or antigen-binding fragment thereof which specifically binds to a second biomarker, said second biomarker being a BMP10-type peptide, FGF23, a BNP-type peptide, GDF15, ANG2, CRP (C-reactive protein), ESM1, or a lipid biomarker, such as Cholesterol or LDL,

wherein optionally, said kit further comprises a detection agent for a third biomarker, said third biomarker being

(i) if a BMP10-type peptide is the second biomarker, CRP, ANG2 or at least one lipid biomarker selected from the group consisting of Cholesterol, TAG (Triglycerides), LDL (Low-density Lipoprotein), HDL (High-density Lipoprotein) and Apolipoprotein A-1, or
(ii) if FGF23 is the second biomarker, a BMP10-type peptide, a BNP-type peptide, CRP, ANG2, or at least one lipid biomarker selected from the group consisting of Cholesterol, TAG (Triglycerides), LDL and HDL, or
(iii) if a BNP-type peptide is the second biomarker, Cholesterol or ANG2,
(iv) if ANG2 is the second biomarker, APOAT or LDL,

wherein the assessment of myocardial infarction is i) the differentiation between type 1 and type 2 myocardial infarction, ii) the diagnosis of type 2 myocardial infarction, or iii) the guidance of myocardial infarction therapy.

12. The method, device, use or kit of any of the preceding claims, wherein

a) the second marker is Cholesterol,
b) the second marker is a BMP-10-type peptide,
c) the second marker is FGF23,
d) the second marker is ANG2,
e) the second marker is a BMP10-type peptide and the third marker is a least one lipid biomarker selected from the group consisting of Cholesterol, TAG, LDL, APOAT and HDL,

f) the second marker is a BMP10-type peptide and the third marker is CRP,
g) the second marker is FGF23 and the third marker is a least one lipid biomarker selected from the group consisting of Cholesterol, TAG, LDL and HDL,
h) the second marker is FGF23 and the third marker is CRP, or
i) the second marker is ANG2 and the third marker is LDL, e.g. for a subject suffering from diabetes.

13. The method, device, use or kit of any of the preceding claims, wherein the BMP10-type peptide is BMP10, proBMP10 or NT-proBMP10, and/or wherein the BNP-type peptide is NT-proBNP, proBNP or BNP.

**Patentansprüche**

1. In-vitro-Verfahren zur Beurteilung von Myokardinfarkt bei einem Individuum:

(a) Bestimmen der Menge eines ersten Biomarkers in einer Probe des Individuums, wobei der erste Biomarker cMyBPC (Cardiac Myosin Binding Protein C) ist;
(b) Bestimmen der Menge eines zweiten Biomarkers in einer Probe des Individuums, wobei der zweite Biomarker ein Lipid-Biomarker, wie etwa Cholesterin oder LDL (Low Density Lipoprotein), ein Peptid vom BMP10-Typ (Peptid vom Bone Morphogenic Protein 10-Typ), FGF23 (Fibroblast Growth Factor 23), ein Peptid vom BNP-Typ (Peptid vom Brain Natriuretic Peptide-Typ), GDF-15 (Growth Differentiation Factor 15), ANG2 (Angiopoietin 2) oder CRP (C-reaktives Protein), ESM1 (Endothelial Cell Specific Molecule 1) ist;
(c) Vergleichen der Mengen der Biomarker mit Referenzen für die Biomarker und/oder Berechnen eines Scores zur Beurteilung von Myokardinfarkt basierend auf den Mengen der Biomarker; und
(d) Beurteilen von Myokardinfarkt basierend auf dem Vergleich und/oder der in Schritt (c) vorgenommenen Berechnung,

wobei die Beurteilung von Myokardinfarkt i) die Unterscheidung zwischen Myokardinfarkt Typ 1 und Typ 2, ii) die Diagnose von Myokardinfarkt Typ 2 oder iii) die Anleitung einer Myokardinfarkttherapie ist.

2. Verfahren nach Anspruch 1, wobei in Schritt (b)

(i) wenn die Menge eines Peptids vom BMP10-Typ als der zweite Biomarker bestimmt wird, das Verfahren ferner das Bestimmen der Menge von mindestens einem Lipid-Biomarker, ausgewählt aus der Gruppe bestehend aus Cholesterin, TAG (Triglyceriden), LDL (Low Density Lipoprotein), HDL (High Density Lipoprotein) und Apolipoprotein A-1, oder der Menge von CRP oder ANG2 als einen dritten Biomarker umfasst; oder
(ii) wenn die Menge von FGF23 als der zweite Biomarker bestimmt wird, das Verfahren ferner das Bestimmen der Menge von mindestens einem Lipid-Biomarker, ausgewählt aus der Gruppe bestehend aus Cholesterin, TAG (Triglyceriden), LDL und HDL, oder einem Peptid vom BMP10-Typ, einem Peptid vom BNP-Typ, CRP oder ANG2 als einen dritten Biomarker umfasst, oder
(iii) wenn die Menge eines Peptids vom BNP-Typ als der zweite Biomarker bestimmt wird, das Verfahren ferner das Bestimmen der Menge von Cholesterin oder ANG2 umfasst, oder
(iv) wenn die Menge von ANG2 als der zweite Biomarker bestimmt wird, das Verfahren ferner das Bestimmen der Menge von oder LDL oder APOAT als einen dritten Biomarker umfasst.

3. Verfahren nach Anspruch 1 oder 2, wobei die Probe von einem Individuum bei Vorstellung in der Notaufnahme erhalten wurde.

4. Verfahren nach einem der Ansprüche 1 bis 3, wobei die Probe eine Blut-, Serum- oder Plasmaprobe ist.

5. Verfahren nach einem der Ansprüche 1 bis 4, wobei das Individuum ein Mensch ist.

6. Computerimplementiertes Verfahren zur Beurteilung von Myokardinfarkt bei einem Individuum, wobei das Verfahren die folgenden Schritte umfasst:

(a) Empfangen eines Werts für die Menge eines ersten Biomarkers in einer Probe des Individuums, wobei der erste Biomarker cMyBPC ist;
(b) Empfangen eines Werts für die Menge eines zweiten Biomarkers in einer Probe des Individuums, wobei der zweite Biomarker ein Peptid vom BMP10-Typ, FGF23, ein Peptid vom BNP-Typ, GDF15, ANG2, CRP (C-reaktives Protein), ESM1 oder ein Lipid-Biomarker, wie etwa Cholesterin oder LDL, ist;

(c) Vergleichen der Werte für die Mengen der Biomarker mit Referenzen für die Biomarker und/oder Berechnen eines Scores zur Beurteilung von Myokardinfarkt basierend auf den Mengen der Biomarker; und

(d) Beurteilen von Myokardinfarkt basierend auf dem Vergleich und/oder der in Schritt (c) vorgenommenen Berechnung,

wobei die Beurteilung von Myokardinfarkt i) die Unterscheidung zwischen Myokardinfarkt Typ 1 und Typ 2, ii) die Diagnose von Myokardinfarkt Typ 2 oder iii) die Anleitung einer Myokardinfarkttherapie ist.

7. Verfahren nach Anspruch 6, wobei in Schritt (b)

(i) wenn der Wert für die Menge eines Peptids vom BMP10-Typ als der zweite Biomarker empfangen wird, das Verfahren ferner das Empfangen eines Werts für die Menge von CRP, ANG2 oder mindestens einem Lipid-Biomarker, ausgewählt aus der Gruppe bestehend aus Cholesterin, TAG (Triglyceriden), LDL (Low Density Lipoprotein), HDL (High Density Lipoprotein) und Apolipoprotein A-1, als einen dritten Biomarker umfasst; oder

(ii) wenn der Wert für die Menge von FGF23 als der zweite Biomarker empfangen wird, das Verfahren ferner das Empfangen eines Werts für die Menge eines Peptids vom BMP10-Typ, eines Peptids vom BNP-Typ, CRP, ANG2 oder mindestens einem Lipid-Biomarker, ausgewählt aus der Gruppe bestehend aus Cholesterin, TAG (Triglyceriden), LDL und HDL, als einen dritten Biomarker umfasst, oder

(iii) wenn der Wert für die Menge eines Peptids vom BNP-Typ als der zweite Biomarker empfangen wird, das Verfahren ferner das Empfangen eines Werts für die Menge von Cholesterin oder ANG2 umfasst, oder

(iv) wenn der Wert für die Menge eines ANG2 als der zweite Biomarker empfangen wird, das Verfahren ferner das Empfangen eines Werts für die Menge von APOAT oder LDL als einen dritten Biomarker umfasst.

8. Vorrichtung zur Beurteilung von Myokardinfarkt bei einem Individuum, wobei die Vorrichtung eine Auswertungseinheit, die eine Datenbank mit gespeicherten Referenzen für einen ersten Biomarker, der cMyBPC ist, und einen zweiten Biomarker umfasst, wobei der zweite Biomarker ein Peptid vom BMP10-Typ, FGF23, ein Peptid vom BNP-Typ, GDF15, ANG2, CRP (C-reaktives Protein), ESM1 oder ein Lipid-Biomarker, wie etwa Cholesterin oder LDL, ist, und einen Datenprozessor umfasst, der Anweisungen zum Durchführen eines Vergleichs der Menge des ersten Biomarkers und des zweiten Biomarkers mit Referenzen, vorzugsweise wie in einem der Ansprüche 1 bis 7 angegeben, und zur Beurteilung von Myokardinfarkt basierend auf dem Vergleich umfasst, wobei die Auswertungseinheit in der Lage ist, Werte für die Mengen der in einer Probe des Individuums bestimmten Biomarker zu empfangen,

wobei die Datenbank gegebenenfalls eine gespeicherte Referenz für einen dritten Biomarker umfasst, wobei der dritte Biomarker

(i) wenn ein Peptid vom BMP10-Typ der zweite Biomarker ist, CRP, ANG2 oder mindestens ein Lipid-Biomarker, ausgewählt aus der Gruppe bestehend aus Cholesterin, TAG (Triglyceriden), LDL (Low Density Lipoprotein), HDL (High Density Lipoprotein) und Apolipoprotein A-1, ist;

(ii) wenn FGF23 der zweite Biomarker ist, ein Peptid vom BMP10-Typ, ein Peptid vom BNP-Typ, CRP, ANG2 oder mindestens ein Lipid-Biomarker, ausgewählt aus der Gruppe bestehend aus Cholesterin, TAG (Triglyceriden), LDL und HDL, ist, oder

(iii) wenn ein Peptid vom BNP-Typ der zweite Biomarker ist, Cholesterin oder ANG2 ist, oder

(iv) wenn ANG2 der zweite Biomarker ist, APOAT oder LDL ist,

wobei die Beurteilung von Myokardinfarkt i) die Unterscheidung zwischen Myokardinfarkt Typ 1 und Typ 2, ii) die Diagnose von Myokardinfarkt Typ 2 oder iii) die Anleitung einer Myokardinfarkttherapie ist.

9. In-vitro-Verwendung von i) einem ersten Biomarker, der cMyBPC ist, und einem zweiten Biomarker, wobei der zweite Biomarker ein Peptid vom BMP10-Typ, FGF23, ein Peptid vom BNP-Typ, GDF15, ANG2, CRP (C-reaktives Protein), ESM1 oder ein Lipid-Biomarker, wie etwa Cholesterin oder LDL, ist, oder ii) mindestens einem Nachweismittel für den ersten Biomarker, mindestens einem Nachweismittel für den zweiten Biomarker, zur Beurteilung von Myokardinfarkt bei einem Individuum, wobei die Beurteilung von Myokardinfarkt i) die Unterscheidung zwischen Myokardinfarkt Typ 1 und Typ 2, ii) die Diagnose von Myokardinfarkt Typ 2 oder iii) die Anleitung einer Myokardinfarkttherapie ist.

10. Verwendung nach Anspruch 9, wobei zusätzlich ein dritter Biomarker oder ein Nachweismittel für einen dritten Biomarker verwendet wird, wobei der dritte Biomarker

(i) wenn ein Peptid vom BMP10-Typ der zweite Biomarker ist, CRP, ANG2 oder mindestens ein Lipid-Biomarker, ausgewählt aus der Gruppe bestehend aus Cholesterin, TAG (Triglyceriden), LDL (Low Density Lipoprotein), HDL (High Density Lipoprotein) und Apolipoprotein A-1, ist,

(ii) wenn FGF23 der zweite Biomarker ist, ein Peptid vom BMP10-Typ, ein Peptid vom BNP-Typ, CRP, ANG2 oder mindestens ein Lipid-Biomarker, ausgewählt aus der Gruppe bestehend aus Cholesterin, TAG (Triglyceriden), LDL und HDL, ist,

(iii) wenn ein Peptid vom BNP-Typ der zweite Biomarker ist, Cholesterin oder ANG2 ist, oder

(iv) wenn ANG2 der zweite Biomarker ist, APOAT oder LDL ist.

11. Kit zur Beurteilung von Myokardinfarkt bei einem Individuum, wobei der Kit mindestens einen Antikörper oder ein antigenbindendes Fragment davon, der bzw. das spezifisch an einen ersten Biomarker, der cMyBPC ist, bindet, und mindestens einen Antikörper oder ein antigenbindendes Fragment davon, der bzw. das spezifisch an einen zweiten Biomarker bindet, umfasst, wobei der zweite Biomarker ein Peptid vom BMP10-Typ, FGF23, ein Peptid vom BNP-Typ, GDF15, ANG2, CRP (C-reaktives Protein), ESM1 oder ein Lipid-Biomarker, wie etwa Cholesterin oder LDL, ist,

wobei der Kit gegebenenfalls ferner ein Nachweismittel für einen dritten Biomarker umfasst, wobei der dritte Biomarker

(i) wenn ein Peptid vom BMP10-Typ der zweite Biomarker ist, CRP, ANG2 oder mindestens ein Lipid-Biomarker, ausgewählt aus der Gruppe bestehend aus Cholesterin, TAG (Triglyceriden), LDL (Low Density Lipoprotein), HDL (High Density Lipoprotein) und Apolipoprotein A-1, ist, oder

(ii) wenn FGF23 der zweite Biomarker ist, ein Peptid vom BMP10-Typ, ein Peptid vom BNP-Typ, CRP, ANG2 oder mindestens ein Lipid-Biomarker, ausgewählt aus der Gruppe bestehend aus Cholesterin, TAG (Triglyceriden), LDL und HDL, ist, oder

(iii) wenn ein Peptid vom BNP-Typ der zweite Biomarker ist, Cholesterin oder ANG2 ist,

(iv) wenn ANG2 der zweite Biomarker ist, APOAT oder LDL ist,

wobei die Beurteilung von Myokardinfarkt i) die Unterscheidung zwischen Myokardinfarkt Typ 1 und Typ 2, ii) die Diagnose von Myokardinfarkt Typ 2 oder iii) die Anleitung einer Myokardinfarkttherapie ist.

12. Verfahren, Vorrichtung, Verwendung oder Kit nach einem der vorhergehenden Ansprüche, wobei

a) der zweite Marker Cholesterin ist,
b) der zweite Marker ein Peptid vom BMP10-Typ ist,
c) der zweite Marker FGF23 ist,
d) der zweite Marker ANG2 ist,
e) der zweite Marker ein Peptid vom BMP10-Typ ist und der dritte Marker mindestens ein Lipid-Biomarker, ausgewählt aus der Gruppe bestehend aus Cholesterin, TAG, LDL, APOAT und HDL, ist,
f) der zweite Marker ein Peptid vom BMP10-Typ ist und der dritte Marker CRP ist,
g) der zweite Marker FGF23 ist und der dritte Marker mindestens ein Lipid-Biomarker, ausgewählt aus der Gruppe bestehend aus Cholesterin, TAG, LDL und HDL, ist,
h) der zweite Marker FGF23 ist und der dritte Marker CRP ist oder
i) der zweite Marker ANG2 ist und der dritte Marker LDL ist, z.B. für ein Individuum, das an Diabetes leidet.

13. Verfahren, Vorrichtung, Verwendung oder Kit nach einem der vorhergehenden Ansprüche, wobei das Peptid vom BMP10-Typ BMP10, proBMP10 oder NT-proBMP10 ist und/oder wobei das Peptid vom BNP-Typ NT- proBNP oder BNP ist.

**Revendications**

1. Procédé in vitro d'évaluation de l'infarctus du myocarde chez un sujet :

(a) détermination de la quantité d'un premier biomarqueur dans un échantillon du sujet, ledit premier biomarqueur étant cMyBPC (protéine C cardiaque de liaison à la myosine) ;

(b) détermination de la quantité d'un deuxième biomarqueur dans un échantillon du sujet, ledit deuxième biomarqueur étant un biomarqueur lipidique, tel que le cholestérol ou le LDL (lipoprotéine basse densité), un peptide de type BMP10 (peptide de type protéine osseuse morphogénétique 10), le FGF23 (facteur de croissance des fibroblastes 23), un peptide de type BNP (peptide de type peptide cérébral natriurétique), le GDF-15 (facteur de croissance et de différenciation 15), l'ANG2 (angiopoïétine 2) ou la CRP (protéine C réactive), l'ESM1 (molécule 1 spécifique des cellules endothéliales) ;

(c) comparaison des quantités des biomarqueurs à des références pour lesdits biomarqueurs et/ou calcul d'un score pour évaluer l'infarctus du myocarde en se basant sur les quantités des biomarqueurs ; et

(d) évaluation de l'infarctus du myocarde en se basant sur la comparaison et/ou le calcul réalisés à l'étape (c),

dans lequel l'évaluation de l'infarctus du myocarde est i) la différenciation entre un infarctus du myocarde de type 1 et de type 2, ii) le diagnostic d'infarctus du myocarde de type 2, ou iii) l'orientation d'une thérapie contre l'infarctus du myocarde.

2.  Procédé selon la revendication 1, dans lequel dans l'étape (b)

    (i) si la quantité d'un peptide de type BMP10 est déterminée comme étant le deuxième biomarqueur, le procédé comprend en outre la détermination de la quantité d'au moins un biomarqueur lipidique choisi dans le groupe constitué par le cholestérol, les TAG (triglycérides), le LDL (lipoprotéine basse densité), le HDL (lipoprotéine haute densité) et l'apolipoprotéine A-1 ou la quantité de CRP ou d'ANG2 comme étant un troisième biomarqueur ; ou

    (ii) si la quantité de FGF23 est déterminée comme étant le deuxième biomarqueur, le procédé comprend en outre la détermination de la quantité d'au moins un biomarqueur lipidique choisi dans le groupe constitué par le cholestérol, les TAG (triglycérides), le LDL et le HDL, ou un peptide de type BMP10, un peptide de type BNP, la CRP ou l'ANG2 en tant que troisième biomarqueur, ou

    (iii) si la quantité d'un peptide de type BNP est déterminée comme étant le deuxième biomarqueur, le procédé comprend en outre la détermination de la quantité de cholestérol ou d'ANG2, ou

    (iv) si la quantité d'ANG2 est déterminée comme étant le deuxième biomarqueur, le procédé comprend en outre la détermination de la quantité de LDL ou d'APOAT comme étant un troisième biomarqueur.

3.  Procédé selon la revendication 1 ou 2, dans lequel l'échantillon a été obtenu auprès d'un sujet lors de la présentation au service des urgences.

4.  Procédé selon l'une quelconque des revendications 1 à 3, dans lequel ledit échantillon est un échantillon de sang, de sérum ou de plasma.

5.  Procédé selon l'une quelconque des revendications 1 à 4, dans lequel le sujet est humain.

6.  Procédé mis en œuvre par ordinateur pour évaluer l'infarctus du myocarde chez un sujet, ledit procédé comprenant les étapes de :

    (a) réception d'une valeur pour la quantité d'un premier biomarqueur dans un échantillon du sujet, ledit premier biomarqueur étant cMyBPC ;

    (b) réception d'une valeur pour la quantité d'un deuxième biomarqueur dans un échantillon du sujet, ledit deuxième biomarqueur étant un peptide de type BMP10, le FGF23, un peptide de type BNP, le GDF15, l'ANG2, la CRP (protéine C réactive), l'ESM1 ou un biomarqueur lipidique, tel que le cholestérol ou le LDL ;

    (c) comparaison des valeurs pour les quantités des biomarqueurs à des références pour lesdits biomarqueurs et/ou le calcul d'un score pour évaluer l'infarctus du myocarde en se basant sur les quantités des biomarqueurs ; et

    (d) évaluation de l'infarctus du myocarde en se basant sur la comparaison et/ou le calcul réalisés à l'étape (c),

dans lequel l'évaluation de l'infarctus du myocarde est i) la différenciation entre un infarctus du myocarde de type 1 et de type 2, ii) le diagnostic d'infarctus du myocarde de type 2, ou iii) l'orientation d'une thérapie contre l'infarctus du myocarde.

7.  Procédé selon la revendication 6, dans lequel dans l'étape (b)

    (i) si la valeur pour la quantité d'un peptide de type BMP10 est reçue comme étant le deuxième biomarqueur, le procédé comprend en outre la réception d'une valeur pour la quantité de CRP, d'ANG2 ou d'au moins un biomarqueur lipidique choisi dans le groupe constitué par le cholestérol, les TAG (triglycérides), le LDL (lipoprotéine basse densité), le HDL (lipoprotéine haute densité) et l'apolipoprotéine A-1 comme étant un troisième biomarqueur ; ou

(ii) si la valeur pour la quantité de FGF23 est reçue comme étant le deuxième biomarqueur, le procédé comprend en outre la réception d'une valeur pour la quantité d'un peptide de type BMP10, d'un peptide de type BNP, de CRP, d'ANG2 ou d'au moins un biomarqueur lipidique choisi dans le groupe constitué par le cholestérol, les TAG (triglycérides), le LDL et le HDL comme étant un troisième biomarqueur, ou

(iii) si la valeur pour la quantité d'un peptide de type BNP est reçue comme étant le deuxième biomarqueur, le procédé comprend en outre la réception d'une valeur pour la quantité de cholestérol ou d'ANG2, ou

(iv) si la valeur pour la quantité d'ANG2 est reçue comme étant le deuxième biomarqueur, le procédé comprend en outre la réception d'une valeur pour la quantité d'APOAT ou de LDL en tant que troisième biomarqueur.

8. Dispositif pour l'évaluation de l'infarctus du myocarde chez un sujet, ledit dispositif comprenant une unité d'évaluation comprenant une base de données avec des références stockées pour un premier biomarqueur qui est cMyBPC et un deuxième biomarqueur, ledit deuxième biomarqueur étant un peptide de type BMP10, le FGF23, un peptide de type BNP, le GDF15, l'ANG2, la CRP (protéine C réactive), l'ESM1 ou un biomarqueur lipidique, tel que le cholestérol ou le LDL, et un processeur de données comprenant des instructions pour réaliser une comparaison de la quantité du premier biomarqueur et du deuxième biomarqueur à des références, de préférence, comme spécifié dans l'une quelconque des revendications 1 à 7 et pour évaluer l'infarctus du myocarde en se basant sur la comparaison, ladite unité d'évaluation étant capable de recevoir des valeurs pour les quantités des biomarqueurs déterminés dans un échantillon du sujet,

dans lequel, éventuellement, ladite base de données comprend une référence stockée pour un troisième biomarqueur, ledit troisième biomarqueur étant

(i) si un peptide de type BMP10 est le deuxième biomarqueur, la CRP, l'ANG2 ou au moins un biomarqueur lipidique choisi dans le groupe constitué par le cholestérol, les TAG (triglycérides), le LDL (lipoprotéine basse densité), le HDL (lipoprotéine haute densité) et l'apolipoprotéine A-1 ;

(ii) si le FGF23 est le deuxième biomarqueur, un peptide de type BMP10, un peptide de type BNP, la CRP, l'ANG2 ou au moins un biomarqueur lipidique choisi dans le groupe constitué par le cholestérol, les TAG (triglycérides), le LDL et le HDL, ou

(iii) si un peptide de type BNP est le deuxième biomarqueur, le cholestérol ou l'ANG2, ou

(iv) si l'ANG2 est le deuxième biomarqueur, l'APOAT ou le LDL,

dans lequel l'évaluation de l'infarctus du myocarde est i) la différenciation entre un infarctus du myocarde de type 1 et de type 2, ii) le diagnostic d'infarctus du myocarde de type 2, ou iii) l'orientation d'une thérapie contre l'infarctus du myocarde.

9. Utilisation in vitro de i) un premier biomarqueur qui est cMyBPC et un deuxième biomarqueur, ledit deuxième biomarqueur étant un peptide de type BMP10, le FGF23, un peptide de type BNP, le GDF15, l'ANG2, la CRP (protéine C réactive), l'ESM1 ou un biomarqueur lipidique, tel que le cholestérol ou le LDL, ou ii) au moins un agent de détection pour ledit premier biomarqueur, au moins un agent de détection pour ledit deuxième biomarqueur, pour évaluer l'infarctus du myocarde chez un sujet, dans laquelle l'évaluation de l'infarctus du myocarde est i) la différenciation entre un infarctus du myocarde de type 1 et de type 2, ii) le diagnostic d'infarctus du myocarde de type 2, ou iii) l'orientation d'une thérapie contre l'infarctus du myocarde.

10. Utilisation selon la revendication 9, dans laquelle un troisième biomarqueur ou un agent de détection pour un troisième biomarqueur est utilisé en plus, ledit troisième biomarqueur étant

(i) si un peptide de type BMP10 est le deuxième biomarqueur, la CRP, l'ANG2 ou au moins un biomarqueur lipidique choisi dans le groupe constitué par le cholestérol, les TAG (triglycérides), le LDL (lipoprotéine basse densité), le HDL (lipoprotéine haute densité) et l'apolipoprotéine A-1,

(ii) si le FGF23 est le deuxième biomarqueur, un peptide de type BMP10, un peptide de type BNP, la CRP, l'ANG2 ou au moins un biomarqueur lipidique choisi dans le groupe constitué par le cholestérol, les TAG (triglycérides), le LDL et le HDL,

(iii) si un peptide de type BNP est le deuxième biomarqueur, le cholestérol ou l'ANG2, ou

(iv) si l'ANG2 est le deuxième biomarqueur, l'APOAT ou le LDL.

11. Kit d'évaluation de l'infarctus du myocarde chez un sujet, ledit kit comprenant au moins un anticorps, ou un fragment de liaison à l'antigène de celui-ci qui se lie spécifiquement à un premier biomarqueur qui est cMyBPC et au moins un

EP 4 493 937 B1

anticorps, ou un fragment de liaison à l'antigène de celui-ci qui se lie spécifiquement à un deuxième biomarqueur, ledit deuxième biomarqueur étant un peptide de type BMP10, le FGF23, un peptide de type BNP, le GDF15, l'ANG2, la CRP (protéine C réactive), l'ESM1 ou un biomarqueur lipidique, tel que le cholestérol ou le LDL,

dans lequel éventuellement, ledit kit comprend en outre un agent de détection pour un troisième biomarqueur, ledit troisième biomarqueur étant

(i) si un peptide de type BMP10 est le deuxième biomarqueur, la CRP, l'ANG2 ou au moins un biomarqueur lipidique choisi dans le groupe constitué par le cholestérol, les TAG (triglycérides), le LDL (lipoprotéine basse densité), le HDL (lipoprotéine haute densité) et l'apolipoprotéine A-1, ou
(ii) si le FGF23 est le deuxième biomarqueur, un peptide de type BMP10, un peptide de type BNP, la CRP, l'ANG2 ou au moins un biomarqueur lipidique choisi dans le groupe constitué par le cholestérol, les TAG (triglycérides), le LDL et le HDL, ou
(iii) si un peptide de type BNP est le deuxième biomarqueur, le cholestérol ou l'ANG2,
(iv) si l'ANG2 est le deuxième biomarqueur, l'APOAT ou le LDL,

dans lequel l'évaluation de l'infarctus du myocarde est i) la différenciation entre un infarctus du myocarde de type 1 et de type 2, ii) le diagnostic d'infarctus du myocarde de type 2, ou iii) l'orientation d'une thérapie contre l'infarctus du myocarde.

12. Procédé, dispositif, utilisation ou kit selon l'une quelconque des revendications précédentes, dans lesquels

a) le deuxième marqueur est le cholestérol,
b) le deuxième marqueur est un peptide de type BMP-10,
c) le deuxième marqueur est le FGF23,
d) le deuxième marqueur est l'ANG2,
e) le deuxième marqueur est un peptide de type BMP10 et le troisième marqueur est au moins un biomarqueur lipidique choisi dans le groupe constitué par le cholestérol, les TAG, le LDL, l'APOAT et le HDL,
f) le deuxième marqueur est un peptide de type BMP10 et le troisième marqueur est la CRP,
g) le deuxième marqueur est le FGF23 et le troisième marqueur est au moins un biomarqueur lipidique choisi dans le groupe constitué par le cholestérol, les TAG, le LDL et le HDL,
h) le deuxième marqueur est le FGF23 et le troisième marqueur est la CRP, ou
i) le deuxième marqueur est l'ANG2 et le troisième marqueur est le LDL, par ex. pour un sujet souffrant du diabète.

13. Procédé, dispositif, utilisation ou kit selon l'une quelconque des revendications précédentes, dans lesquels le peptide de type BMP10 est BMP10, proBMP10 ou NT-proBMP10, et/ou dans lesquels le peptide de type BNP est NT-proBNP, proBNP ou BNP.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20120213782 A, Susan-Resiga
- WO 2020035605 A1
- WO 2021165465 A1
- WO 02089657 A
- WO 02083913 A
- WO 2004099253 A1
- WO 9906445 A
- WO 0070051 A
- WO 2005113585 A
- WO 2003073464 A

### Non-patent literature cited in the description

- **UPTON PD et al.** protective effects of BMP10 in atherosclerotic plaques. *J Cell Sci*, 2020
- **THYGESEN K et al.** *Circulation*, 2018, vol. 138, e618-e651
- **DEFILIPPIS et al.** *Circulation*, 2019, vol. 140, 1661-1678
- **THYGESEN et al.** *Eur Heart J*, 2018, vol. 40 (3), 246-247
- **MCCARTHY et al.** *JAMA*, 2018, vol. 320 (5), 433
- **COLLINSON et al.** *ACC*, 18 May 2016
- **SMILOWITZ et al.** *Coronary Artery Disease*, 2018, vol. 29 (1), 46-52
- **NAGELE.** *Circulation*, 2020, vol. 2020 (141), 1431-1433
- **NESTELBERGER et al.** *JAMA Cardiol.*, 2021
- **COLLET JP et al.** *Eur Heart J*, 2021, vol. 42, 1289-1367
- **THYGESEN et al.** *Eur J Heart*, 2019, vol. 40 (3), 237-269
- **DOWDY ; WEARDEN.** Statistics for Research. John Wiley & Sons, 1983
- **COLLET JP et al.** ESC Guidelines for the management of acute coronary syndromes in patients presenting without persistent ST-segment elevation. *Eur Heart J.*, 29 August 2020, 575
- **SUSAN-RESIGA et al.** *J Biol Chem.*, 01 July 2011, vol. 286 (26), 22785-94
- **YADIN et al.** *CYTOGFR*, 2016, vol. 27 (2016), 13-34
- **SHIMADA et al.** *PNAS*, 2001, vol. 98 (11), 6500-6505
- **BONOW.** New Insights into the cardiac natriuretic peptides. *Circulation*, 1996, vol. 93, 1946-1950
- **SCHELLENBERGER et al.** *Arch Biochem Biophys*, 2006, 451
- **HALFINGER et al.** *Clinical Chemistry*, 2017, vol. 63 (1), 359-368
- **MAISONPIERRE et al.** *Science*, 1997, vol. 277, 55-60
- **HROMAS.** *Biochim Biophys Acta*, 1997, vol. 1354, 40-44
- **LAWTON.** *Gene*, 1997, vol. 203, 17-26
- **YOKOYAMA-KOBAYASHI.** *J Biochem (Tokyo)*, 1997, vol. 122, 622-626
- **PARALKAR.** *J Biol Chem*, 1998, vol. 273, 13760-13767
- **BOTTNER.** *Gene*, 1999, vol. 237, 105-111
- **BAEK.** *Mol Pharmacol*, 2001, vol. 59, 901-908
- **MORRISH.** *Placenta*, 1996, vol. 17, 431-441
- **WOO et al.** *J. Biol. Chem.*, 1985, vol. 260 (24), 13384-13388
- **YEH.** *Circulation*, 2004, vol. 2004 (109), 11-11, 11-14
- **UHLÉN M. et al.** *Science*, 2015, vol. 347 (6220), 1260419
- **BECHARD D et al.** *J Biol Chem*, 2001, vol. 276 (51), 48341-48349
- **LASSALE P. et al.** *J. Biol. Chem.*, 1996, vol. 271, 20458-20464
- **ZWEIG.** *Clin. Chem.*, 1993, vol. 39, 561-577
- **NESTELBERGER et al.** *JAMA Cardiol.*, 2021, vol. 6 (7), 771-780
- **THYGESEN K et al.** *Eur Heart J.*, 2019, vol. 40, 226